(19) Europäisches Patentamt / European Patent Office / Office européen des brevets

(11) **EP 4 302 704 A1**

(12) **EUROPEAN PATENT APPLICATION**
published in accordance with Art. 153(4) EPC

(43) Date of publication:
**10.01.2024 Bulletin 2024/02**

(21) Application number: **22763329.4**

(22) Date of filing: **02.03.2022**

(51) International Patent Classification (IPC):
**A61B 10/00** (2006.01)    **A61B 5/0245** (2006.01)
**A61B 5/11** (2006.01)

(52) Cooperative Patent Classification (CPC):
**A61B 5/0245; A61B 5/11; A61B 10/00**

(86) International application number:
**PCT/JP2022/008871**

(87) International publication number:
**WO 2022/186275 (09.09.2022 Gazette 2022/36)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: 05.03.2021 US 202163156936 P
29.10.2021 JP 2021178089

(71) Applicant: **The University of
Electro-Communications
Chofu-shi, Tokyo 182-8585 (JP)**

(72) Inventors:
• **TAKADAMA, Keiki
  Chofu-shi, Tokyo 182-8585 (JP)**
• **MATSUDA, Naoya
  Chofu-shi, Tokyo 182-8585 (JP)**

(74) Representative: **Hautier IP - MC/EP
17, avenue Albert II
C/O The Office & Co - L'ALBU
98000 Monaco (MC)**

(54) **ALZHEIMER-TYPE DEMENTIA DETERMINATION DEVICE, ALZHEIMER-TYPE DEMENTIA DETERMINATION METHOD, AND PROGRAM**

(57) A dementia judgement device includes an acquisition unit configured to acquire information about a heart rate, a first estimation unit configured to estimate a circadian rhythm of the heart rate from the information about the heart rate, a second estimation unit configured to estimate a non-circadian rhythm of the heart rate, which is a rhythm different from the circadian rhythm of the heart rate, and a judgement unit configured to judge whether a subject has Alzheimer-type dementia according to a signal amplitude of the estimated circadian rhythm of the heart rate and a signal amplitude of the estimated non-circadian rhythm of the heart rate.

FIG. 2

**Description**

[Technical Field]

The present invention relates to an Alzheimer-type dementia judgement device, an Alzheimer-type dementia judgement method, and a program.
Priority is claimed on 63/156,936, filed in the United States on March 5, 2021, and Japanese Patent Application No. 2021-178089, filed October 29, 2021, the content of which is incorporated herein by reference.

[Background Art]

In recent years, the number of dementia patients has been increasing. About half of cases of dementia are of Alzheimer-type dementia. No complete prevention method or fundamental treatment method for Alzheimer-type dementia has been found, and since it takes about 10 to 20 years for the initial symptoms to appear, treatment is often difficult when Alzheimer-type dementia is found. Therefore, early detection and early treatment of Alzheimer-type dementia have become important issues.
For Alzheimer-type dementia judgement, for example, a test using a Mini-Mental State Examination (MMSE) test sheet, which is one neuropsychological test performed when dementia is suspected, is used. In this judgement method, subjects answer the 11 test questions asked by questioners one by one. The time required for the test is about 10 to 15 minutes.
Elderly subjects with Alzheimer-type dementia with sleep disorders have a higher frequency of awakening in the middle of night or early in the morning due to shallow sleep than elderly subjects with non-Alzheimer-type dementia with sleep disorders. Based on this trend, a method in which a mattress-type sensor is used to observe an individual's sleep status (the sleep time and the number of awakenings due to sensor pressurization) over a long period of time from the viewpoint of sleep disorders in Alzheimer-type dementia, and to analyze association with Alzheimer-type dementia has been proposed (refer to Non-Patent Document 1).

[Citation List]

[Non-Patent Document]

[Non-Patent Document 1]

H. Nikamalfard, et al., "A sleep pattern analysis and visualization system to support people with early dementia', The fifth International Conference on Pervasive Computing Technologies for Healthcare and Workshops, pp. 510-513, 2011.

[Summary of Invention]

[Technical Problem]

However, in a screening test based on verbal questions such as MMSE, regular testing is difficult because it has a psychological burden on test subjects. There is a problem that it is difficult to find opportunities for conducting an MMSE in the early stage of dementia which conditions seem to be the same as those of healthy subjects.
In the method described in Non-Patent Document 1, it is difficult to decisively distinguish between sleep statuses of elderly subjects with Alzheimer-type dementia and non-Alzheimer-type dementia. Therefore, it is still uncertain to judge Alzheimer-type dementia according to only the sleep time and the number of awakenings.
The present invention has been made to address the above problems, and an object of the present invention is to provide an Alzheimer-type dementia judgement device, an Alzheimer-type dementia judgement method, and a program through which it is possible to distinguish between Alzheimer-type dementia patients and healthy subjects with less burden on test subjects.

[Solution to Problem]

In order to achieve the above object, an Alzheimer-type dementia judgement device according to one aspect of the present invention includes an acquisition unit configured to acquire information about a heart rate; a first estimation unit configured to estimate a circadian rhythm of the heart rate from the information about the heart rate; a second estimation unit configured to estimate a non-circadian rhythm of the heart rate, which is a rhythm different from the circadian rhythm of the heart rate; and a judgement unit configured to judge whether a subject has Alzheimer-type dementia according to a signal amplitude of the estimated circadian rhythm of the heart rate and a signal amplitude of the estimated non-circadian rhythm of the heart rate.

In order to achieve the above object, an Alzheimer-type dementia judgement device according to one aspect of the present invention includes an acquisition unit configured to acquire information about a heart rate; an estimation unit configured to estimate a circadian rhythm of the heart rate from the information about the heart rate; and a judgement unit configured to judge whether a subject has Alzheimer-type dementia according to a sine wave component and a cosine wave component of a signal of the estimated circadian rhythm of the heart rate.

In order to achieve the above object, an Alzheimer-type dementia judgement device according to one aspect of the present invention includes an acquisition unit configured to acquire information about a heart rate; a first estimation unit configured to estimate a circadian rhythm of the heart rate from the information about the heart rate; and a judgement unit configured to judge whether a subject has Alzheimer-type dementia according to a sine wave component of a signal of the estimated circadian rhythm of the heart rate.

In order to achieve the above object, an Alzheimer-type dementia judgement method according to one aspect of the present invention includes, acquiring, by an acquisition unit, information about a heart rate; estimating, by a first estimation unit, a circadian rhythm of the heart rate from the information about the heart rate; estimating, by a second estimation unit, a non-circadian rhythm of the heart rate, which is a rhythm different from the circadian rhythm of the heart rate; and determining, by a judgement unit, whether a subject has Alzheimer-type dementia according to a signal amplitude of the estimated circadian rhythm of the heart rate and a signal amplitude of the estimated non-circadian rhythm of the heart rate.

In order to achieve the above object, an Alzheimer-type dementia judgement method according to one aspect of the present invention includes acquiring, by an acquisition unit, information about a heart rate; estimating, by an estimation unit, a circadian rhythm of the heart rate from the information about the heart rate; and determining, by a judgement unit, whether a subject has Alzheimer-type dementia according to a sine wave component and a cosine wave component of a signal of the estimated circadian rhythm of the heart rate.

In order to achieve the above object, an Alzheimer-type dementia judgement method according to one aspect of the present invention includes, acquiring, by an acquisition unit, information about a heart rate; estimating, by a first estimation unit, a circadian rhythm of the heart rate from the information about the heart rate; and determining, by a judgement unit, whether a subject has Alzheimer-type dementia according to a sine wave component of a signal of the estimated circadian rhythm of the heart rate.

In order to achieve the above object, a program according to one aspect of the present invention causes a computer to: acquire information about a heart rate; estimate a circadian rhythm of the heart rate from the information about the heart rate; estimate a non-circadian rhythm of the heart rate, which is a rhythm different from the circadian rhythm; and judge whether a subject has Alzheimer-type dementia according to a signal amplitude of the estimated circadian rhythm of the heart rate and a signal amplitude of the estimated non-circadian rhythm of the heart rate.

In order to achieve the above object, a program according to one aspect of the present invention causes a computer to: acquire information about a heart rate; estimate a circadian rhythm of the heart rate from the information about the heart rate; and judge whether a subject has Alzheimer-type dementia according to a sine wave component and a cosine wave component of a signal of the estimated circadian rhythm of the heart rate.

In order to achieve the above object, a program according to one aspect of the present invention causes a computer to: acquire information about a heart rate; estimate a circadian rhythm of the heart rate from the information about the heart rate; and judge whether a subject has Alzheimer-type dementia according to a sine wave component of a signal of the estimated circadian rhythm of the heart rate.

[Advantageous Effects of Invention]

According to embodiments of the present invention, it is possible to distinguish between Alzheimer-type dementia patients and healthy subjects with less burden on test subjects.

[Brief Description of Drawings]

FIG. 1 is a diagram showing an example of melatonin secretion volume of healthy elderly subjects and Alzheimer-type dementia patients.
FIG. 2 is a diagram showing a configuration example of a dementia judgement device according to a first embodiment.
FIG. 3 is a diagram showing an example of an estimated circadian rhythm $f_{CR}(t)$ and an estimated non-circadian rhythm $f_{NCR}(t)$ estimated by a fitting.

FIG. 4 is an enlarged diagram of a sleep time period of FIG. 3.

FIG. 5 is a diagram for illustrating heart rate data and a method of calculating an estimated heart rate $f_k(t)$ for 24 hours and an ak average.

FIG. 6 is a flowchart of processing procedures of the dementia judgement device according to the first embodiment.

FIG. 7 is an image diagram of a mattress-type sensor system and an image diagram showing a position of a subject that is used for the sensor.

FIG. 8 is a diagram showing accumulation of each subject group for a predicted amplitude ratio $r_p$ of a circadian rhythm in each data piece.

FIG. 9 is a diagram showing judgement results obtained by a predicted amplitude ratio $r_p$ of the sine wave and the cosine wave of the circadian rhythm and the non-circadian rhythm according to the first embodiment.

FIG. 10 is a diagram for explaining an estimation method for 8 to 40 hours.

FIG. 11 is a diagram showing a configuration example of a dementia judgement device according to a second embodiment.

FIG. 12 is a diagram illustrating examples of a stable heart rate and an unstable heart rate.

FIG. 13 is a flowchart of processing procedures of the dementia judgement device according to the second embodiment.

FIG. 14 is a diagram showing examples of evaluation results according to the second embodiment.

FIG. 15 is a diagram showing examples of the instability ratio of the sine wave and the cosine wave of Alzheimer-type dementia patients, healthy young subjects, healthy middle-aged subjects, and healthy elderly subjects.

FIG. 16 is a diagram for illustrating a method according to a third embodiment.

FIG. 17 is a diagram showing a configuration example of a dementia judgement device according to the third embodiment.

FIG. 18 is a flowchart of processing procedures of the dementia judgement device according to the third embodiment.

FIG. 19 is a diagram showing examples of evaluation results according to the third embodiment.

[Description of Embodiments]

Hereinafter, embodiments of the present invention will be described with reference to the drawings. Here, in the drawings used for the following description, scales of members may be appropriately changed in order for the members to be made to have recognizable sizes.

[Overview]

An overview of each embodiment will be described. In each embodiment, the subject's heart rate is used to estimate a circadian rhythm, and Alzheimer-type dementia patients and healthy elderly subjects are distinguished according to the estimated circadian rhythm. Here, the circadian rhythm is controlled by a biological clock typified by sleep and awakening, and its period is about 24 hours.

(Overview of principle)

An overview of a principle in which Alzheimer-type dementia patients and healthy subjects are distinguished based on a circadian rhythm will be described.

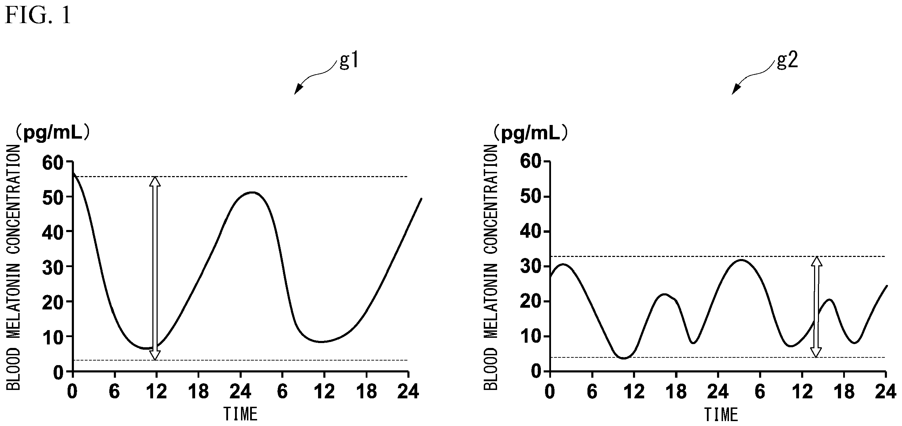

FIG. 1 is a diagram showing an example of melatonin secretion volume of healthy elderly subjects and Alzheimer-type dementia patients. A graph g1 shows a melatonin secretion volume of healthy elderly subjects in each hour, a graph g2 shows a melatonin secretion volume of Alzheimer-type dementia patients, and in the graphs g1 and g2, the horizontal axis represents time (hours), and the vertical axis represents melatonin secretion volume (pg/ml). As shown in FIG. 1, the healthy elderly subjects have a stable circadian rhythm based on the melatonin secretion volume. On the other hand, the Alzheimer-type dementia patients do not clearly express the circadian rhythm based on the melatonin secretion volume (that is, are unstable), and the melatonin secretion volume is lower than that of healthy elderly subjects. This is a disturbance of the rhythm of abnormal secretion of melatonin, which is one symptom of Alzheimer-type dementia, and is a circadian rhythm disorder.

When a circadian rhythm disorder appears, the circadian rhythm becomes unstable, and the circadian characteristics of the biorhythm of the core body temperature disappears. It is known that the heart rate also shows circadian characteristics (for example, refer to Reference 1).

Reference 1; M. Massin, et al., "Circadian rhythm of heartrate and heartrate variability", Archives of Disease in Child-hood, Vol. 83, pp. 179-182, 2000.

Based on the above two points, we hypothesized that the biorhythm of the heart rate is disturbed, and circadian characteristics disappears by the circadian rhythm disorder due to circadian rhythm disturbance. Note that validation results will be described below. Therefore, in the embodiment, the Alzheimer-type dementia is judged based on the circadian rhythm that appears in the heart rate.

<First embodiment>

In the present embodiment, from heart rate data, a circadian rhythm with a period of about 24 hours and a non-circadian rhythm which is a biorhythm other than the circadian rhythm (non-24-hour period) are estimated with a trigonometric function according to maximum likelihood estimation, in order to compare these circadian rhythms. Then, in the present embodiment, for example, a ratio of the circadian rhythm amplitude/non-circadian rhythm amplitude (circadian rhythm amplitude ratio) is calculated from the estimated amplitude, and Alzheimer-type dementia is judged based on the calculated ratio.

[Configuration of dementia judgement device]

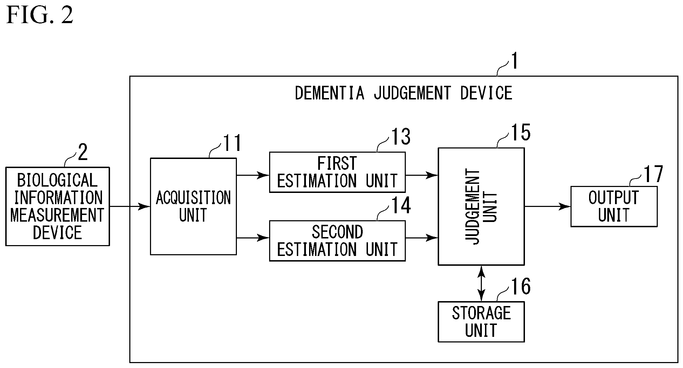

FIG. 2 is a diagram showing a configuration example of a dementia judgement device according to the present embodiment. As shown in FIG. 2, a dementia judgement device 1 includes an acquisition unit 11, a first estimation unit 13, a second estimation unit 14, a judgement unit 15, a storage unit 16, and an output unit 17.
During measurement, a biological information measurement device 2 is connected to the dementia judgement device 1.
The biological information measurement device 2 is, for example, a mattress-type sensor, a smart watch including a sensor for detecting biological information, or a wearable terminal including a sensor for detecting biological information. The biological information measurement device 2 measures data related to at least a heart rate during sleep of a subject, and outputs the measured data to the dementia judgement device 1. In the following description, an example in which the biological information measurement device 2 is, for example, a mattress-type sensor, will be described. In the case of a mattress-type sensor, data related to the heart rate is biological vibration data.
Here, the dementia judgement device 1 and the biological information measurement device 2 are connected in a wired or wireless manner. Here, the biological information measurement device 2 includes a communication unit or interface for data output with the dementia judgement device 1.
The acquisition unit 11 acquires data related to the heart rate (hereinafter referred to as "heart rate data") from the biological information measurement device 2.
The first estimation unit 13 estimates a circadian rhythm of the heart rate (hereinafter referred to as a "circadian rhythm") from the heart rate data.
The second estimation unit 14 estimates a non-circadian rhythm (hereinafter referred to as a "non-circadian rhythm") of the heart rate, which is a different from the circadian rhythm of the heart rate, from the heart rate data.
The judgement unit 15 judges whether the subject has Alzheimer-type dementia based on the ratio of the signal amplitude of the estimated circadian rhythm of the heart rate and the signal amplitude of the estimated non-circadian rhythm of the heart rate.
The storage unit 16 stores formulas, threshold values and the like used by the judgement unit 15. The storage unit 16 may store data related to the heart rate during sleep.
The output unit 17 outputs the judgement result judged by the judgement unit 15 to an external device. Examples of external devices include an image display device, a printing device, a personal computer, a tablet terminal, a smartphone, and a dedicated terminal. Here, the output unit 17 includes a communication unit or interface.

[Processes of first estimation unit and second estimation unit]

Next, processes performed by the first estimation unit 13 and the second estimation unit 14 will be described.
The first estimation unit 13 and the second estimation unit 14 perform maximum likelihood estimation on the heart rate using, for example, Real-time Sleep Stage Estimation (RSSE) (refer to Reference 2). The RSSE performs maximum likelihood estimation on the heart rate, which is represented by a trigonometric function composed of a plurality of frequency components, with respect to the subject's biological vibration data during sleep obtained from the biological information measurement device 2. Then, the RSSE is a method of estimating 6 sleep stages (awakening, REM sleep, non-REM sleep from 1 to 4) from the obtained estimated heart rate.
Reference 2; T. Harada, et al., "Real-Time Sleep Stage Estimation from Biological Data with Trigonometric Function Regression Model", AAAI Spring Symposium, pp.348-353, 2016.
The estimated heart rate $f(t)$ is represented, based on the period set $L=\{2^{14}, ..., 2^2, 2^1\}$[seconds], as shown in the following Formula (1), using a sine wave and a cosine wave of the period set L and their coefficients $a_{l,i}$ ($l \in L$, $i \in \{s, c\}$(s is a coefficient of a sine wave, and c is a coefficient of a cosine wave)), and a constant term C. The first estimation unit 13 and the second estimation unit 14 estimate the coefficient $a_{i,i}$ and the constant term C based on the heart rate data during sleep and output the estimated heart rate f(t).
[Math. 1]

$$f(t) = \sum_{l \in L} \{ a_{l,s} \cos m_l t + a_{l,c} \sin m_l t \} + C$$

$$where \quad m_t = \frac{2\pi}{l} \qquad \cdots (1)$$

A likelihood function used for maximum likelihood estimation is defined as in the following Formula (2) using the raw data HR(t) of the heart rate at time t.
[Math. 2]

$$J = \frac{1}{T} \sum_{t=1}^{T} \{ HR(t) - f(t) \}^2 + \frac{\lambda}{N} \sum_{l \in L} \left( a_{l,c}^2 + a_{l,s}^2 \right) \qquad \cdots (2)$$

$$where \quad N = |L|, \quad T = |HR|$$

In Formula (2), the first term $1/T \cdot \Sigma_{t=1}^{T} \{HR(t)-f(t)\}^2$ fits the estimated heart rate f(t) to HR(t). The second term $\lambda/|L| \cdot \Sigma_{l \in L} \{a_{l,c}^2 + a_{l,s}^2\}$ reduces overfitting of $a_{l,i}$. Here, $\lambda$ is the weight of the second term, and is, for example, 1.0. N represents the total number of parameters to be estimated and is |L|. The first estimation unit 13 and the second estimation unit 14 calculate the coefficient $a_{l,i}$ and the constant term C from a derivation formula obtained by partially differentiating parameters of the likelihood function J , using the last data (t=T) from the first data (t=0) of the heart rate data, as shown in the following Formula (3).. However, the first estimation unit 13 and the second estimation unit 14 skip the above calculation in the times when HR(t) does not exist.
[Math. 3]

$$\frac{\partial J}{\partial a_s} = -\frac{2}{T} \sum_{t=1}^{T} \cos m_l t \{ HR(t) - f(t) \} + \frac{2\lambda}{N} a_s = 0$$

$$\frac{\partial J}{\partial a_c} = -\frac{2}{T} \sum_{t=1}^{T} \sin m_l t \{ HR(t) - f(t) \} + \frac{2\lambda}{N} a_c = 0 \qquad \cdots (3)$$

$$\frac{\partial J}{\partial C} = -\frac{2}{T} \sum_{t=1}^{T} \{ HR(t) - f(t) \} = 0$$

The first estimation unit 13 and the second estimation unit 14 replace the period set L used for estimating the estimated heart rate f(t) with a circadian period set and a non-circadian rhythm period set, and estimate respective amplitudes.

As the circadian rhythm period set (hereinafter referred to as "$L_{CR}$") having a period of about 24 hours, $L_{CR}$ is defined as, for example, {25, 24, 23} [hours], that is around 24 hours. As the non-circadian rhythm period set (hereinafter referred to as "$L_{NCR}$"), $L_{NCR}$ is defined as, for example, {12.5, 12, 11.5}[hours], that is around 12 hours. Here, the above $L_{CR}$ is an example, and may be, for example, {26, 25, 24, 23, 22} or {24.5, 24, 23.5}. Similarly, the above $L_{NCR}$ is an example, and may be, for example, {13, 12.5, 12, 11.5, 11} or {12.25, 12, 11.75}.

The reasons for using 12 hours as the non-circadian rhythm period are the following Reasons I to III.

Reason I; since the circadian rhythm period does not always fall within the range of 23 to 25 hours depending on the person and may be shorter than 23 hours, it may be difficult to be distinguished from the circadian rhythm if its period is longer than 12 hours.

Reason II; using a period shorter than 12 hours easily fits to complex heart rate fluctuations and may be difficult to correctly estimate its amplitude.

Reason III; as long as the method using a circadian rhythm amplitude ratio is used, it is desirable to avoid using waves with periodically different properties. For example, it is easy to explain medically when a rhythm with no circadian characteristics such as an ultradian rhythm (biorhythm with a period of about 90 minutes) is defined as a non-circadian rhythm, but its periodic properties are greatly different.

$L_{CR}$ and $L_{NCR}$ are set as L in Formula (1), and estimated heart rates of the circadian rhythm $f_{CR}(t)$ and the non-circadian rhythm $f_{NCR}(t)$ are obtained as in the following Formula (4).

[Math. 4]

$$f_{CR}(t) = \sum_{l \in L_{CR}} \left\{ a_{l,s} \cos m_l t + a_{l,c} \sin m_l t \right\} + C$$

$$f_{NCR}(t) = \sum_{l \in L_{NCR}} \left\{ a_{l,s} \cos m_l t + a_{l,c} \sin m_l t \right\} + C \quad \cdots (4)$$

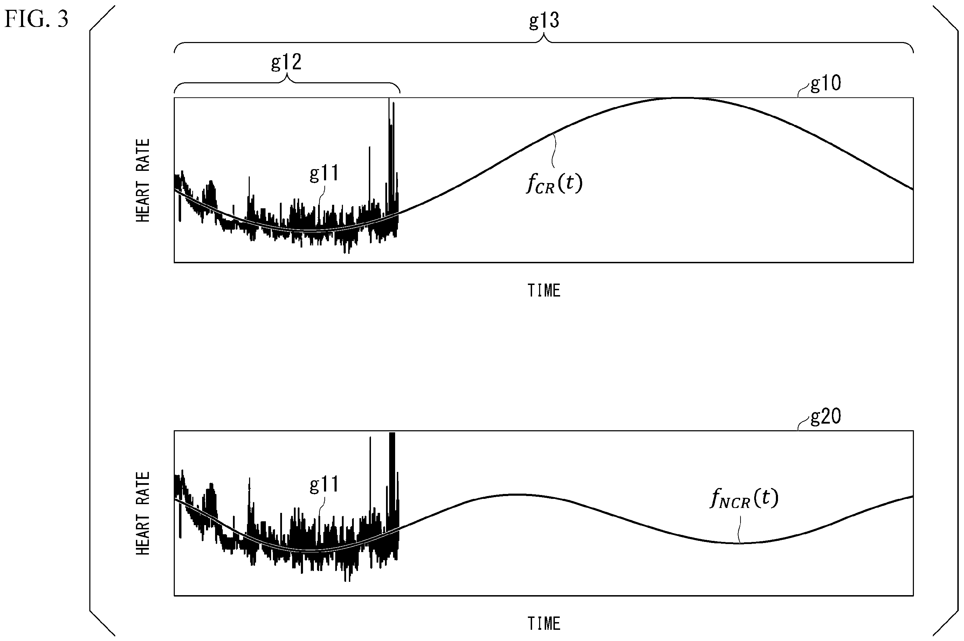

FIG. 3 is a diagram showing an example of the estimated circadian rhythm $f_{CR}(t)$ and the estimated non-circadian rhythm $f_{NCR}(t)$. A graph g10 shows the heart rate (g11) and the estimated circadian rhythm $f_{CR}(t)$, and a graph g20 shows the heart rate (g11) and the estimated circadian rhythm $f_{NCR}(t)$. In this example, the subject's sleep time starts at about 23:30 and ends at about 6:30. The heart rate data is acquired during this sleep time. Here, an absolute value amplitude ratio of the sine wave and the cosine wave of the circadian rhythm and the non-circadian rhythm, which will be described below, is calculated using heart rate data in the range (g12) actually measured during the sleep time. On the other hand, a predicted amplitude ratio of the sine wave and the cosine wave of the circadian rhythm and the non-circadian rhythm is estimated in a range (g13) of about 24 hours or about 12 hours using heart rate data actually measured during the sleep time. Here, the predicted amplitude ratio is calculated by a ratio of the amplitude of the circadian rhythm and the amplitude of the non-circadian rhythm, which are estimated, using heart rate data acquired during the sleep time.

The estimated circadian rhythm $f_{CR}(t)$ in the graph g10 is estimated as the following Formula (5), and the estimated non-circadian rhythm $f_{NCR}(t)$ in the graph g20 is estimated as the following Formula (6).

[Math. 5]

$$f_{CR}(t) = -9.9 \sin\left(\frac{2\pi t}{23.0 \cdot 60^2}\right) - 10.6 \sin\left(\frac{2\pi t}{24.0 \cdot 60^2}\right)$$

$$-11.2 \sin\left(\frac{2\pi t}{25.0 \cdot 60^2}\right) - 6.0 \cos\left(\frac{2\pi t}{23.0 \cdot 60^2}\right)$$

$$-4.2 \cos\left(\frac{2\pi t}{24.0 \cdot 60^2}\right) - 2.9 \cos\left(\frac{2\pi t}{25.0 \cdot 60^2}\right) + 90.3$$

$$\cdots (5)$$

[Math. 6]

$$f_{NCR}(t) = +2.9 \sin\left(\frac{2\pi t}{11.5 \cdot 60^2}\right) + 3.1 \sin\left(\frac{2\pi t}{12.0 \cdot 60^2}\right)$$

$$+4.9 \sin\left(\frac{2\pi t}{12.5 \cdot 60^2}\right) - 12.5 \cos\left(\frac{2\pi t}{11.5 \cdot 60^2}\right)$$

$$-3.7 \cos\left(\frac{2\pi t}{12.0 \cdot 60^2}\right) - 7.0 \cos\left(\frac{2\pi t}{12.5 \cdot 60^2}\right) + 67.5$$

$$\cdots (6)$$

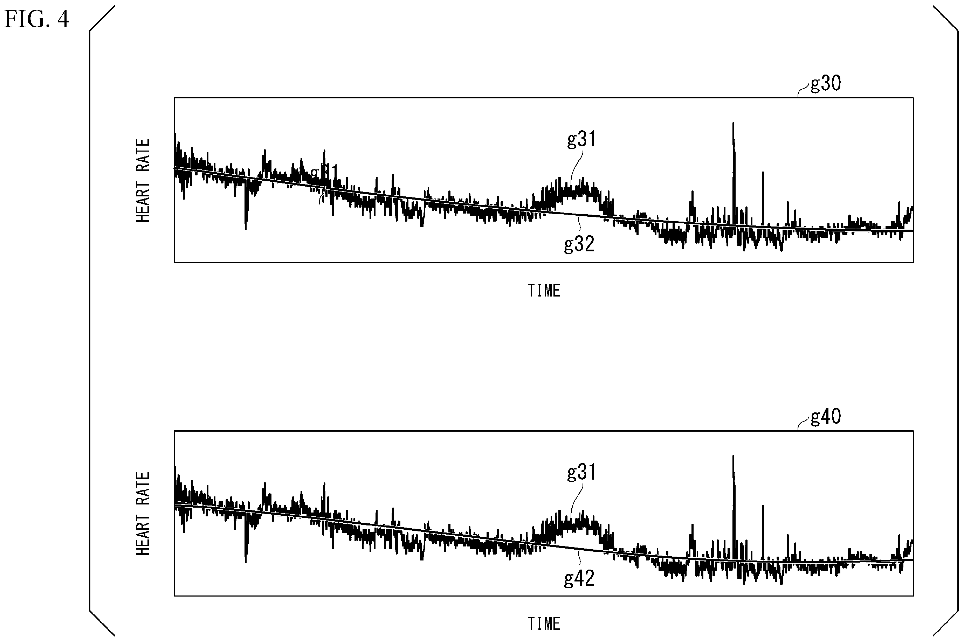

FIG. 4 is an enlarged diagram of the sleep time period of FIG. 3. A graph g30 shows the heart rate data and the estimated heart rate $f_{CR}(t)$ in the period set $L_{CR}$ of the circadian rhythm. A graph g40 shows the heart rate data and the estimated heart rate $f_{NCR}(t)$ in the period set $L_{NCR}$ of the non-circadian rhythm. In the graphs 30 and g40, the vertical axis represents the heart rate, and the horizontal axis represents time. The line g11 represents heart rate data, the line g32 represents the estimated heart rate $f_{CR}(t)$ composed of waves of the $L_{CR}$ period set, and the line g32 represents the estimated heart rate $f_{NCR}(t)$ composed of waves of the $L_{NCR}$ period set.

(Process of judgement unit)

The judgement unit 15 calculates the amplitudes of the circadian rhythm and the non-circadian rhythm as $a_{CR}$ and $a_{NCR}$ from the estimated heart rate $f_{CR}(t)$ and the estimated heart rate $f_{NCR}(t)$ in order to calculate a circadian rhythm amplitude ratio r used for determining Alzheimer-type dementia. Hereinafter, in this specification, the amplitude of the circadian rhythm will be referred to as an "amplitude $a_{CR}$." Hereinafter, in this specification, the amplitude of the non-circadian rhythm will be referred to as an "amplitude $a_{NCR}$."

The judgement unit 15 calculates the ratio r of the amplitude of the circadian rhythm and the amplitude of the non-circadian rhythm as in the following Formula (7).

[Math. 7]

$$ r = \frac{\overline{a_{CR}}}{\overline{a_{NCR}}} \quad \cdots (7) $$

The following Formula (8) is obtained by calculating the absolute value average of the coefficients ai, i of all waves without distinguishing between the sine wave and the cosine wave of $L^k$. $r_A$ (ratio by Absolute average) is defined as the circadian rhythm absolute value amplitude ratio of the amplitude $a_{CR}$ of the circadian rhythm and the amplitude $a_{NCR}$ of the non-circadian rhythm. Hereinafter, in this specification, Formula (8) will be referred to as an "average $a_k$."

[Math. 8]

$$ r_A = \frac{\overline{a_{CR}}}{\overline{a_{NCR}}} $$

$$ \overline{a_k} = \frac{1}{2|L|} \sum_{l \in L_k} \left( \left| a_{l,s} \right| + \left| a_{l,c} \right| \right) where \ k \in \{CR, NCR\} \quad \cdots (8) $$

Here, $r_A$ as the absolute value amplitude ratio of the circadian rhythm calculated from the average $a_k$ calculated as the absolute average value of the coefficients l, i of both the sine wave and the non-sine wave.

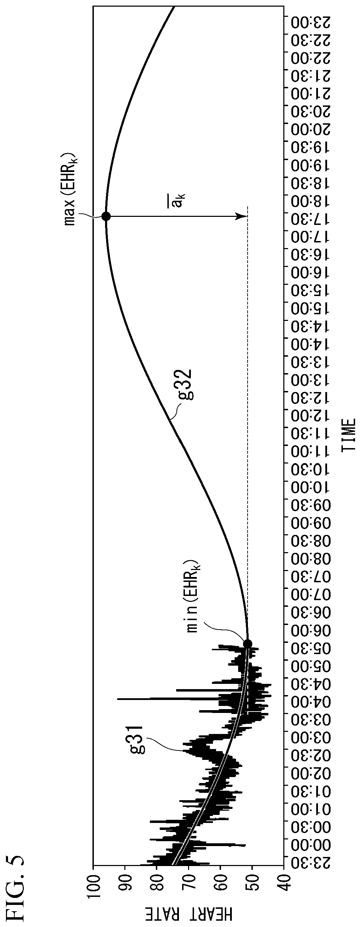

The judgement unit 15 calculates the estimated heart rate $f_{CR}(t)$ and $f_{NCR}(t)$ (range of t=0 to 86,400 seconds (=24 hours)) for one day rates. The judgement unit 15 calculates the difference between the maximum value and the minimum value (=predicted amplitude) as the average $a_k$. Specifically, it is represented by the following Formula (9) and can be shown as in FIG. 5.

[Math. 9]

$$r_P = \frac{\overline{a_{CR}}}{\overline{a_{NCR}}}$$

$$\overline{a_k} = \max\left(EHR_k\right) - \min\left(EHR_k\right)$$

$$where \quad EHR_k\left(t\right) = f_k\left(t\right)$$

$$\left(0 \le t \le 86400 = 24 \cdot 60 \cdot 60,\ k \in \left\{CR, NCR\right\}\right)$$

$$\cdots (9)$$

In Formula (9), $EHR_k$ is an estimated value of the heart rate for one day obtained by substituting t=0 to 86,400 seconds (=1 day) for $f_k$(t). As shown in Formula (9), the judgement unit 15 obtains the maximum value and the minimum value from this $EHR_k$, and calculates the difference between the maximum value and the minimum value as the average $a_k$.

FIG. 5 is a diagram for illustrating heart rate data and a method of calculating the estimated heart rate $f_k$(t) for 24 hours and an average $a_k$. The vertical axis represents the heart rate, the horizontal axis represents time, the line g31 represents the heart rate data, and the line g32 represents transition of the estimated heart rate $f_k$(t) for one day.
$r_P$ (ratio by Predicted amplitude) is defined as the predicted amplitude ratio of the amplitude $a_{CR}$ of the circadian rhythm and the amplitude $a_{NCR}$ of the non-circadian rhythm.

When the circadian rhythm amplitude ratio of the heart rate (=circadian rhythm amplitude/non-circadian rhythm amplitude) is lower than the threshold value, the judgement unit 15 judges that the circadian rhythm is unstable, and judges that the subject has Alzheimer-type dementia. On the other hand, when the amplitude ratio is higher than the threshold value, the judgement unit 15 judges that the circadian rhythm is stable and judges that the subjects has no Alzheimer-type dementia.

Specifically, it is assumed that the amplitude $a_{CR}$ is large when the circadian rhythm is stable, while the amplitude $a_{CR}$ is small when the circadian rhythm is unstable. It is assumed that the amplitude $a_{NCR}$ is large when the circadian rhythm is unstable while the amplitude $a_{NCR}$ is small when the circadian rhythm is stable.

The judgement unit 15 judges that the circadian rhythm is unstable when both of the absolute value amplitude ratio $r_A$ and the predicted amplitude ratio $r_P$ of circadian rhythms are smaller than the threshold value of 1.0. It judges that the circadian rhythm is stable when both of the absolute value amplitude ratio $r_A$ and the predicted amplitude ratio $r_P$ of circadian rhythms are larger than the threshold value of 1.0.

The judgement unit 15 judges that the subject has Alzheimer-type dementia when the circadian rhythm is evaluated as unstable according to two amplitude ratios. This is because it is expected to improve judgement accuracy of Alzheimer-type dementia by combining these features which stability and instability of the circadian rhythm differ depending on the method of calculating the amplitude $a_{CR}$ and the amplitude $a_{NCR}$.

The features of each circadian rhythm amplitude ratio are summarized as follows.

The absolute value amplitude ratio $r_A$ has a feature of calculating an amplitude while ignoring phase information of each wave by utilizing an absolute value of wave coefficients $a_{l,i}$. Thereby, for example, it is possible to distinguish whether all the coefficients ai, i are small due to a low amplitude of $f_k$(t) or the final amplitude is low due to the occurrence of cancellation of waves (positive and negative coefficients).

On the other hand, the predicted amplitude ratio $r_P$ has a feature of calculating the amplitude of the estimated heart rate $f_k$(t).

[Processing procedure example]

Next, the processing procedure example of the dementia judgement device will be described.

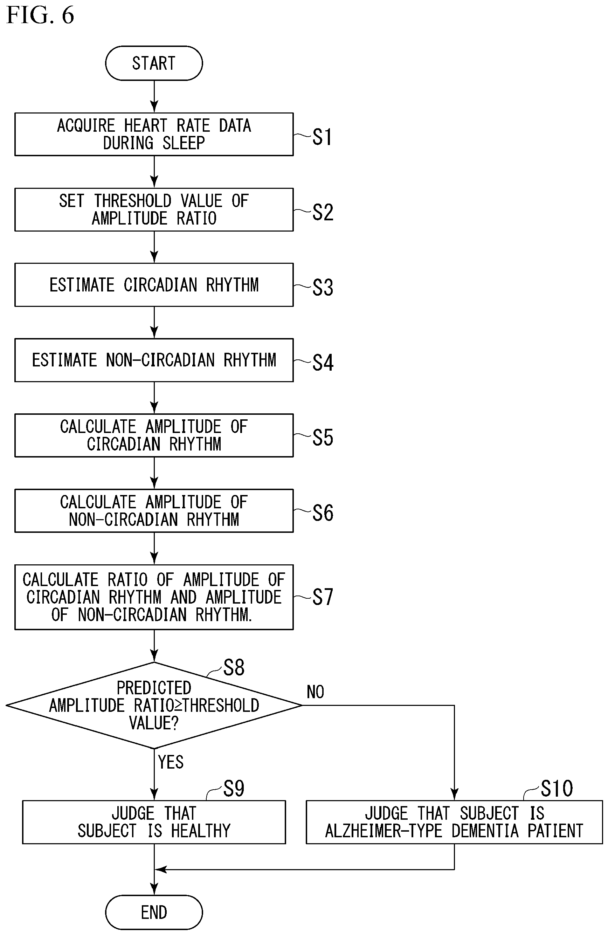

FIG. 6 is the flowchart of processing procedures of the dementia judgement device according to the present embodiment.

(Step S1) The acquisition unit acquires the heart rate data during sleep.
(Step S2) The judgement unit 15 sets the threshold value of the amplitude ratio $r_{amp}$.
(Step S3) The first estimation unit 13 estimates the circadian rhythm from the heart rate data.

(Step S4) The second estimation unit 14 estimates the non-circadian rhythm from the heart rate data.

(Step S5) The judgement unit 15 calculates the signal amplitude of the estimated circadian rhythm.

(Step S6) The judgement unit 15 calculates the signal amplitude of the estimated non-circadian rhythm.

(Step S7) The judgement unit 15 calculates the predicted amplitude ratio of the signal amplitude of the circadian rhythm and the signal amplitude of the non-circadian rhythm.

(Step S8) The judgement unit 15 judges whether the predicted amplitude ratio is equal to or larger than the threshold value $r_{amp}$ (for example, the threshold value $r_{amp}$ is set to 1.0). When the judgement unit 15 judges that the predicted amplitude ratio is equal to or larger than the threshold value $r_{amp}$ (YES in Step S8), the process proceeds to Step S9. When the judgement unit 15 judges that the predicted amplitude ratio is less than the threshold value $r_{amp}$ (NO in Step S8), the process proceeds to Step S10.

(Step S9) The judgement unit 15 judges that the subject is healthy person. After the process, the judgement unit 15 terminates the process.

(Step S10) The judgement unit 15 judges that the subject is an Alzheimer-type dementia patient. After the process, the judgement unit 15 terminates the process.

[Evaluation results]

Next, the results obtained by evaluating the judgement of Alzheimer-type dementia using the absolute value amplitude ratio of the sine wave and the cosine wave of the estimated circadian rhythm and the estimated non-circadian rhythm described above and the judgement of Alzheimer-type dementia using the predicted amplitude ratio of the sine wave and the cosine wave of the circadian rhythm and the non-circadian rhythm using the subject's data will be described.

In the evaluation, for each of four subject groups, the effectiveness of the two judgement methods were verified from the Alzheimer-type dementia judgement rate based on the absolute value amplitude ratio $r_A$ and the predicted amplitude ratio $r_P$, and the comprehensive Alzheimer-type dementia judgement rate ($r_A \cap r_P$) based on two amplitude ratios. Here, the four subject groups were categorized as elderly Alzheimer-type dementia patients, healthy (=non-Alzheimer-type dementia patient) elderly subjects, healthy middle-aged subjects, and healthy young subjects. The threshold value was set to 1.0. Here, in the evaluation, the human subject experiment was approved by the ethics committee institution to which the inventors belong, and all subjects signed the consent form.

The following data were analyzed as the targets in this evaluation: the heart rate data during sleep (n=17) for 17 days for one Alzheimer-type dementia patient in the dementia care facility, the heart rate data during sleep (n=6) for several days for three healthy elderly subjects (60s to 70s), the heart rate data during sleep (n=14) for several days for 10 healthy middle-aged subjects (40s to 50s), and the heart rate data during sleep (n=10) for several days for 8 healthy young subjects (20s to 30s).

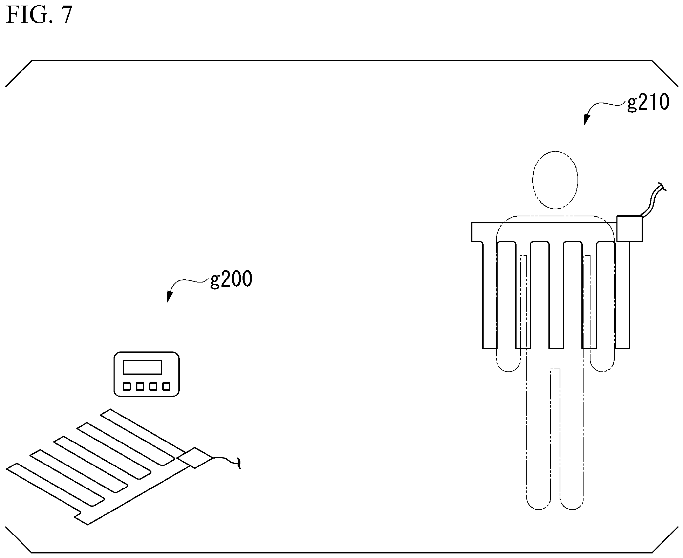

Here, the heart rate data was measured using a mattress-type sensor Emfit (commercially available from Emfit). FIG. 7 is the image diagram of a mattress-type sensor system and the image diagram showing a position of a subject for the sensor. The image diagram g200 shows the mattress-type sensor system, which includes a set of a sensor, a data transmitter and etc. The image diagram g210 shows a position of a subject for the sensor. The mattress-type sensor is installed, for example, under the bed mattress at a position corresponding to the upper body of the subject.

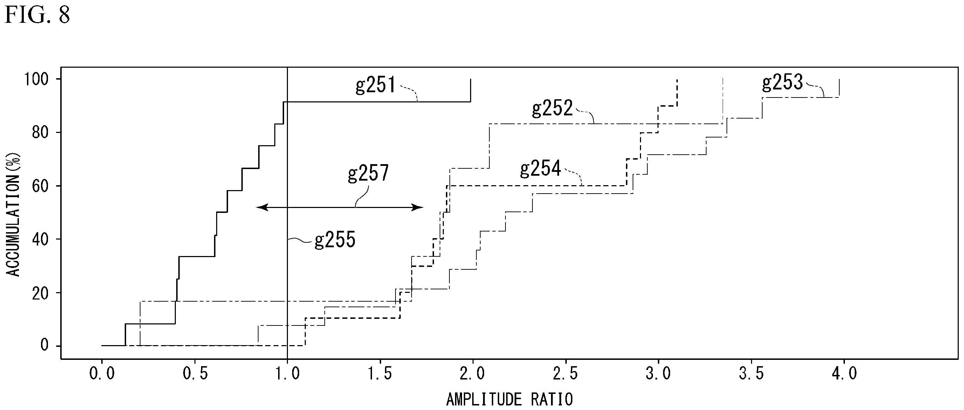

FIG. 8 is a diagram showing accumulation of each subject group for the predicted amplitude ratio $r_p$ of the circadian rhythm for each data. In FIG. 8, the horizontal axis represents the circadian rhythm amplitude ratio, and the vertical axis represents accumulation (0 to 100%). In the data accumulation, the line g251 represents the Alzheimer-type dementia patients, the line g252 represents the healthy elderly subjects, the line g253 represents the healthy middle-aged subjects, and the line g254 represents the healthy young subjects.

The point on each accumulation line represents the judgement percentage of Alzheimer-type dementia patients [%] (=the order when the ratio is sorted in descending order within each subject group or the total number of data pieces within the subject group) when the threshold value is set to the value in the horizontal axis. The line g255 represents that the threshold value is set to 1.0, and the point on the left side of this line (a ratio value is lower than 1.0) is data judged as an Alzheimer-type dementia patient. That is, the accumulation of intersections between the line of each subject group and the line g255 is the judgement rate of Alzheimer-type dementia when the threshold value is set to 1.0.

The correct answer rate for evaluation according to the absolute value amplitude ratio $r_A$ of the sine wave and the cosine wave of the circadian rhythm and the non-circadian rhythm was 100.0% for the Alzheimer-type dementia patients, 16.7% for the healthy elderly subjects, 71.4% for the healthy middle-aged subjects, and 80.0% for the young subjects. According to this evaluation method, it is possible to judge Alzheimer-type dementia patients.

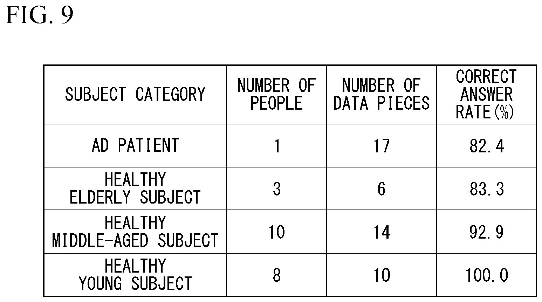

FIG. 9 is a diagram showing the judgement results obtained by the predicted amplitude ratio $r_P$ of the sine wave and the cosine wave of the circadian rhythm and the non-circadian rhythm according to the present embodiment.

As shown in FIG. 9, the correct answer rate for judgement using the predicted amplitude ratio $r_P$ of the sine wave and the cosine wave of the circadian rhythm and the non-circadian rhythm were 82.4% for the Alzheimer-type dementia patients, 83.3% for the healthy elderly subjects, 92.9% for the healthy middle-aged subjects, and 100.0% for the healthy young subjects.

As indicated by the arrow g257 in FIG. 8, in the case of judgement using the predicted amplitude ratio of the sine wave and the cosine wave of the circadian rhythm and the non-circadian rhythm, the Alzheimer-type dementia patients, healthy elderly subjects, healthy middle-aged subjects and healthy young subjects are separated according to the threshold value of 1.0, and thus the elderly subjects can be separated into the Alzheimer-type dementia patients and the healthy subjects.

Thus, the Alzheimer-type dementia judgement using the predicted amplitude ratio of the sine wave and the cosine wave of the circadian rhythm and the non-circadian rhythm was highly accurate.

(Modified example)

In the above example, the example in which a circadian rhythm (about 24 hours) and a non-circadian rhythm (about 12 hours) were separately estimated has been described, but the present invention is not limited thereto. In the above example, the example in which an amplitude of a circadian rhythm and an amplitude of a non-circadian rhythm were estimated has been described, but the present invention is not limited thereto.

For example, a circadian rhythm and a non-circadian rhythm may be separately estimated, and evaluation may be performed based on the average of coefficients of the sine wave and the cosine wave instead of the amplitude of the circadian rhythm and the amplitude of the non-circadian rhythm.

Alternatively, a circadian rhythm and a non-circadian rhythm may be separately estimated, and evaluation may be performed based on the coefficient of composite waves of the sine wave and the cosine wave instead of the amplitude of the circadian rhythm and the amplitude of the non-circadian rhythm.

Alternatively, a circadian rhythm and a non-circadian rhythm are simultaneously estimated using the following Formula (10), the amplitude of the circadian rhythm and the amplitude of the non-circadian rhythm are estimated, and evaluation may be performed based on the amplitude ratio. Here, in Formula (10), the first term represents the circadian rhythm, and the second term represents the non-circadian rhythm.

[Math. 10]

$$f(t) = \sum_{l=23\,\text{hours}}^{25\,\text{hours}} \left\{ a_{l,s} \cos\left(\frac{2\pi t}{l}\right) + a_{l,c} \sin\left(\frac{2\pi t}{l}\right) \right\}$$
$$+ \sum_{l=23\,\text{hours}}^{25\,\text{hours}} \left\{ a_{l/2,s} \cos\left(\frac{2\pi t}{l/2}\right) + a_{l,c} \sin\left(\frac{2\pi t}{l/2}\right) \right\} + C$$
$$\cdots \quad (1\,0)$$

Alternatively, a circadian rhythm and a non-circadian rhythm are simultaneously estimated using Formula (10), the amplitude of the circadian rhythm and the amplitude of the non-circadian rhythm are estimated, and evaluation may be performed based on the average of coefficients of the sine wave and the cosine wave instead of the amplitude of the circadian rhythm and the amplitude of the non-circadian rhythm.

Alternatively, a circadian rhythm and a non-circadian rhythm are simultaneously estimated using Formula (10), and evaluation may be performed based on the coefficient of composite waves of the sine wave and the cosine wave instead of the amplitude of the circadian rhythm and the amplitude of the non-circadian rhythm.

Alternatively, in consideration of the sleep time, using the following Formula (11) and the following Formula (12), for example, a circadian rhythm and a non-circadian rhythm are simultaneously estimated for 8 to 40 hours, and evaluation may be performed based on the average of coefficients of the sine wave and the cosine wave instead of the amplitude of the circadian rhythm and the amplitude of the non-circadian rhythm. Specifically, the coefficient of the ratio of the average $a_{CR}$ (Formula (11)) and the average $a_{NCR}$ (Formula (12)) is taken as the absolute average of the wave coefficients.

[Math. 11]

$$\overline{a_{CR}} = \sum_{l=8\,\text{hours}}^{40\,\text{hours}} \left( \left| a_{l,s} \right| + \left| a_{l,c} \right| \right) N(l, 24h, 1.0)$$

$$\cdots (1\,1)$$

[Math. 12]

$$\overline{a_{NCR}} = \sum_{l=8\,\text{hours}}^{40\,\text{hours}} \left( \left| a_{l,s} \right| + \left| a_{l,c} \right| \right) \left\{ \max\left( N(l, 24h, 1.0) \right) - N(l, 24h, 1.0) \right\}$$

$$\cdots (1\,2)$$

Here, in Formula (11) and Formula (12), the subscript s represents a sine wave and the subscript c represents a cosine wave. N(L, 24H, 1.0) is a value of a normal distribution $f_{(L)}$ with an average of 24 hours and a variance of 1.0.

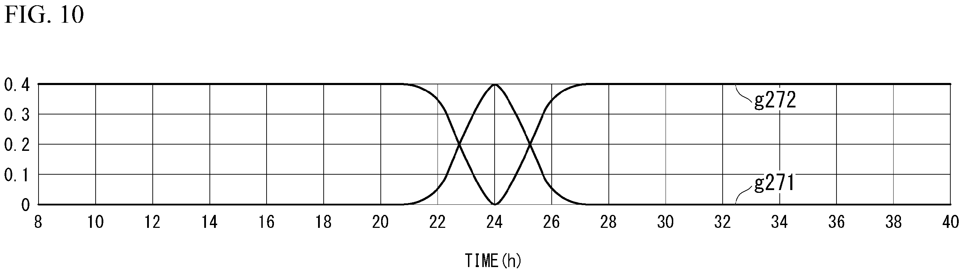

FIG. 10 is a diagram for explaining an estimation method for 8 to 40 hours. The horizontal axis represents time and the vertical axis represents a value of a normal distribution $f_{(L)}$ with a variance of 1. The line g271 represents a normal distribution with an average of 24 hours and a variance of 1.0 (N(24H, 1.0)) and the line g272 represents a normal distribution with an average of 24 hours and a variance of 1.0 (max-N(24H, 1.0)).

Here, in each of the above modified examples, in the case of the average of the sine wave and the cosine wave, an absolute value average of wave coefficients is calculated by the following Formula (13).

[Math. 13]

$$\overline{a_k} = \frac{1}{\left| L_k \right|} \sum_{l \ni L_k} \left( \left| a_{l,s} \right| + \left| a_{l,c} \right| \right) \quad \cdots (1\,3)$$

In each of the above modified examples, in the case of composite waves of the sine wave and the cosine wave, an average of coefficients of composite waves for wave coefficients is calculated by the following Formula (14).

[Math. 14]

$$\overline{a_k} = \frac{1}{\left| L_k \right|} \sum_{l \ni L_k} \left( \sqrt{a_{l,s}^2 + a_{l,c}^2} \right) \quad \cdots (1\,4)$$

In each of the above modified examples, when the amplitude is estimated, the amplitude is calculated from data for one period of waves by the following Formula (15).

[Math. 15]

$$\overline{a_k} = \max\left( \text{Synthetic wave of period } L_k \right)$$

$$- \min\left( \text{Synthetic wave of period } L_k \right) \quad \cdots (1\,5)$$

As described above, in the present embodiment, the first estimation unit 13 estimates a heart rate circadian rhythm from the heart rate, the second estimation unit 14 estimates a non-circadian rhythm of a heart rate, which is a rhythm that is different from the circadian rhythm of the heart rate, and the judgement unit 15 judges whether the subject has Alzheimer-type dementia according to the signal amplitude of the estimated circadian rhythm of the heart rate and the signal amplitude of the estimated non-circadian rhythm of the heart rate.

Thereby, according to the present embodiment, it is possible to distinguish between Alzheimer-type dementia patients and healthy subjects with less burden on test subjects. In the present embodiment, the evaluation results show that the erroneous judgement rate for healthy subjects is low while the judgement rate of Alzheimer-type dementia is also high in the month when the progress of Alzheimer-type dementia in its patients is suspected to be significant.

Here, in the above example, the example using heart rate data has been described as an example of biological information, but the present invention is not limited thereto. The biological information can be, for example, pulse rate data. Here, in the embodiment, "information about a heart rate" corresponds to "heart rate data" or "pulse rate data."

Here, in the above example, the example in which the ratio of the amplitude of the circadian rhythm and the amplitude of the non-circadian rhythm is compared with the threshold value has been described, but the present invention is not limited thereto. The judgement unit 15 may compare the difference between the amplitude of the circadian rhythm and the amplitude of the non-circadian rhythm with the threshold value, and distinguish between Alzheimer-type dementia patients and healthy subjects according to the comparison results.

<Second embodiment>

In the first embodiment, the example in which the threshold value (for example, 1.0) is used for judgement has been described, but in the present embodiment, the example in which the threshold value is not used for judgement will be described. In the present embodiment, an instability ratio of the circadian rhythm estimated from the heart rate is calculated, and it is judged whether the subject has Alzheimer-type dementia according to the value of the calculated instability ratio. Here, the instability ratio of the sine wave is a ratio of the sum of absolute values of coefficients of sine wave components of the circadian rhythm of the heart rate and the sum of coefficients of sine wave components of the estimated circadian rhythm of the heart rate. The instability ratio of the cosine wave is the ratio of the sum of absolute values of coefficients of the cosine wave components of the circadian rhythm of the heart rate and the sum of coefficients of the cosine wave components of the estimated circadian rhythm of the heart rate. Here, the instability ratio does not need to be a sum and can be judged by, for example, a sum multiplication.

[Configuration of dementia judgement device]

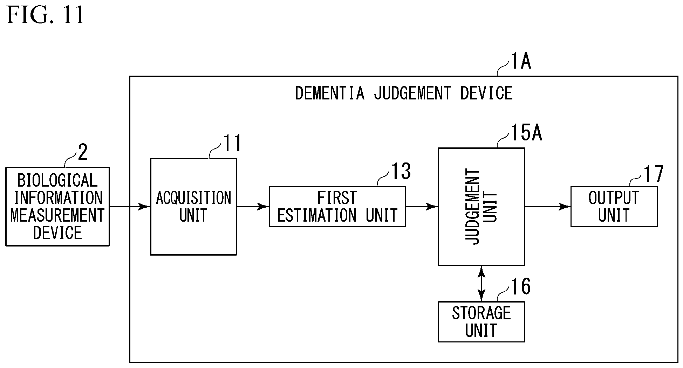

FIG. 11 is the diagram showing the configuration example of the dementia judgement device according to the present embodiment. As shown in FIG. 11, the dementia judgement device 1A includes the acquisition unit 11, the first estimation unit 13 (estimation unit), the judgement unit 15A, the storage unit 16, and the output unit 17.

During measurement, the biological information measurement device 2 is connected to the dementia judgement device 1A.

The first estimation unit 13 estimates a circadian rhythm from heart rate data.

The judgement unit 15A calculates an instability ratio R of the circadian rhythm, and judges whether the subject has Alzheimer-type dementia according to the value of the calculated instability ratio R.

[Stable heart rate and unstable heart rate]

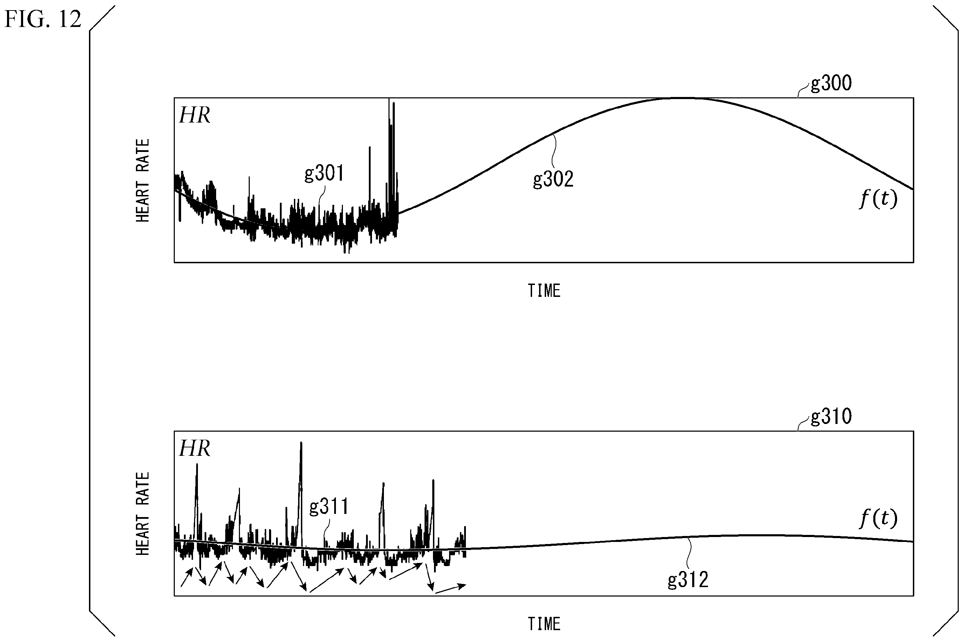

FIG. 12 is the diagram illustrating examples of a stable heart rate and an unstable heart rate. The graph g300 represents the example in which the measured heart rate data of healthy subjects shows a stable circadian rhythm. The graph g310 represents the example in which the measured heart rate data of Alzheimer-type dementia patients shows an unstable circadian rhythm. In the graphs g300 and g310, the horizontal axis represents time while the vertical axis represents heart rate. The lines g301 and g311 represent actually measured values and the lines g302 and g312 represent the estimated heart rate f(t) per day.

The estimated heart rate f(t) in the graph g300 is represented by the following Formula (16) and the estimated heart rate f(t) in the graph g310 is represented by the following Formula (17).

[Math. 16]

$$f(t) = -11.18\sin\left(\frac{2\pi t}{23.0 \cdot 60^2}\right) - 10.61\sin\left(\frac{2\pi t}{24.0 \cdot 60^2}\right)$$

$$-9.88\sin\left(\frac{2\pi t}{25.0 \cdot 60^2}\right) - 2.90\cos\left(\frac{2\pi t}{23.0 \cdot 60^2}\right)$$

$$-4.24\cos\left(\frac{2\pi t}{24.0 \cdot 60^2}\right) - 5.94\cos\left(\frac{2\pi t}{25.0 \cdot 60^2}\right)$$

$$\cdots (1\,6)$$

[Math. 17]

$$f(t) = +0.24\sin\left(\frac{2\pi t}{11.5 \cdot 60^2}\right) - 0.71\sin\left(\frac{2\pi t}{12.0 \cdot 60^2}\right)$$

$$-1.79\sin\left(\frac{2\pi t}{12.5 \cdot 60^2}\right) - 0.87\cos\left(\frac{2\pi t}{11.5 \cdot 60^2}\right)$$

$$+0.26\cos\left(\frac{2\pi t}{12.0 \cdot 60^2}\right) + 1.26\cos\left(\frac{2\pi t}{12.5 \cdot 60^2}\right)$$

$$\cdots (1\,7)$$

In the case of healthy subjects, the heart rate gradually decreases and finally increases as shown in the graph g300, which shows that the estimated heart rate f(t) follows a stable circadian rhythm. In the case of healthy subjects, as shown in Formula (16), the coefficient $a_{i,s}$ of the sine wave and the coefficient $a_{i,c}$ of the cosine wave have the same sign and are sufficiently large.

On the other hand, in the case of Alzheimer-type dementia patients, , the heart rate repeatedly increases and decreases for a certain short period as shown in the graph g310, which shows that the estimated heart rate f(t) follows an unstable circadian rhythm. Compared to the graph g300, the amplitude of the estimated heart rate f(t) is small. In the case of Alzheimer-type dementia patients, as shown in Formula (17), the coefficients $a_{l,i}$ become positive or negative, the waves cancel each other out, and the amplitude of the estimated heart rate f(t) is reduced.

Based on these differences, in the present embodiment, the estimated circadian rhythm stability is evaluated with the numerical value R calculated as in the following Formula (18). Here, Ri is calculated by the absolute value of the ratio. $R_i$ is calculated by the absolute value of the ratio of (i) the absolute value of the coefficients $a_{l,i}$ contained in the denominator and (ii) the simple sum of the coefficients ai, i contained in the denominator. Here, i is s (sine wave) or c (cosine wave). Here, R is the average of $R_s$ of the sine wave and $R_c$ of the cosine wave. Here, R is not limited to the average of $R_s$ of the sine wave and $R_c$ of the cosine wave, and may be, for example, a weighted average obtained by multiplying $R_s$ of the sine wave with a weight.

[Math. 18]

$$R_i = \left|\frac{\sum_{l \in L}\left|a_{l,i}\right|}{\sum_{l \in L} a_{l,i}}\right|$$

$$R = \frac{R_s + R_c}{2} \qquad \cdots (1\,8)$$

When the estimated circadian rhythm is stable, the coefficients tend to have the same sign, and two sums are expected to have the same value. That is, Ri is expected to be 1.0. For example, in the example of the following Formula (19), the judgement unit 15A judges that the rhythm is stable because Ri is 1.0 caused by the same sign coefficients.
[Math. 19]

$$+12.8\cos\left(\frac{2\pi t}{11.5\cdot 60^2}\right)+5.5\cos\left(\frac{2\pi t}{12.0\cdot 60^2}\right)+3.0\cos\left(\frac{2\pi t}{12.5\cdot 60^2}\right)$$

$$\rightarrow \left|\frac{12.8+5.4+3.0}{12.8+5.4+3.0}\right|=1.0$$

$$\cdots (19)$$

In unstable cases in which coefficients tend to have different signs, conversely, the sum of the absolute values is expected to be larger than the sum of values. That is, Ri is expected to be larger than 1.0. For example, in the example of the following Formula (20), the judgement unit 15A judges that the coefficients cancel each other out and the rhythm is unstable because Ri is 1.0 or more caused by the different sign coefficients.
[Math. 20]

$$+26.3\cos\left(\frac{2\pi t}{11.5\cdot 60^2}\right)+3.1\cos\left(\frac{2\pi t}{12.0\cdot 60^2}\right)-24.1\cos\left(\frac{2\pi t}{12.5\cdot 60^2}\right)$$

$$\rightarrow \left|\frac{26.3+3.1+24.1}{26.3+3.1-24.1}\right|>1.0$$

$$\cdots (20)$$

R is an average of $R_0$ of the sine wave and $R_1$ of the cosine wave. Therefore, when the R of an Alzheimer-type dementia patient is 1.0, the judgement unit 15A judges that the subject is a non-Alzheimer-type dementia patient because of the stable circadian rhythm. Here, when R is more than 1.0, the judgement unit 15A judges that the subject is an Alzheimer-type dementia patient because of the unstable heart rate circadian rhythm.

[Processing procedure example]

Next, the processing procedure example of the dementia judgement device will be described.

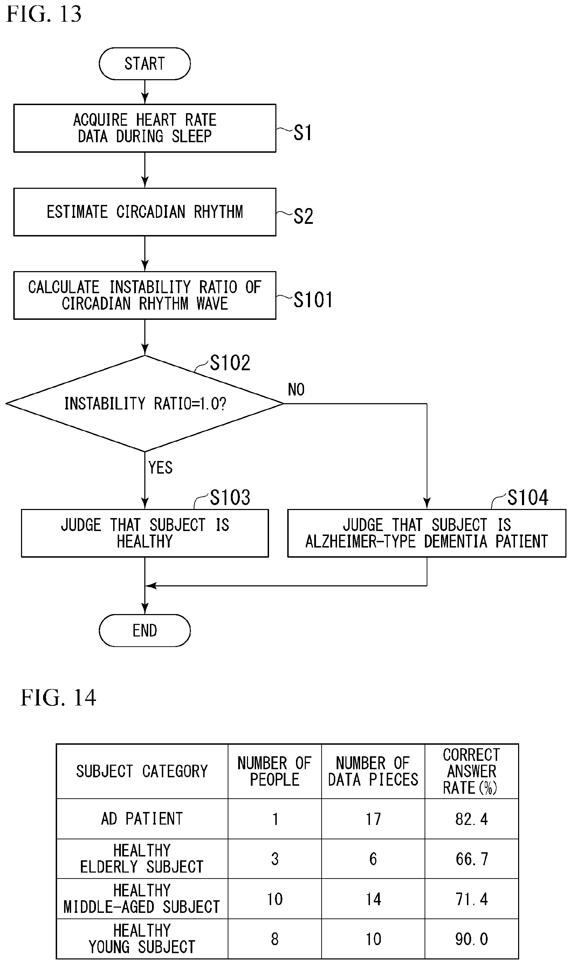

FIG. 13 is the flowchart of processing procedures of the dementia judgement device according to the present embodiment.
(Step S1) The acquisition unit acquires heart rate data during sleep.
(Step S2) The first estimation unit 13 estimates a circadian rhythm from the heart rate data.
(Step S101) The judgement unit 15A calculates an instability ratio R of circadian rhythm waves.
(Step S102) The judgement unit 15A judges whether the instability ratio is 1.0. When the determination unit 15A determine that the instability ratio is 1.0 (YES in Step S102), the process proceeds to Step S103. When the determination unit 15A determine that the instability ratio is not 1.0 (NO in Step S102), the process proceeds to Step S104.
(Step S103) The judgement unit 15A judges that the subject is healthy. After the process, the judgement unit 15A terminates the process.
(Step S104) The judgement unit 15A judges that the subject is an Alzheimer-type dementia patient. After the process, the judgement unit 15A terminates the process.

[Evaluation results]

Next, the evaluation result example will be described. Here, in the evaluation, the human subject experiment was approved by the ethics committee institution to which the inventors belong, and all subjects signed the consent form. The evaluation criteria employ Alzheimer-type dementia detection accuracy (Alzheimer-type dementia detection rate of Alzheimer-type dementia patients and non-Alzheimer-type dementia detection rate of healthy subjects).

FIG. 14 is the diagram showing the examples of evaluation results according to the present embodiment. As shown in FIG. 14, according to the present embodiment, the correct answer rate of the Alzheimer-type dementia patients was 82.4%, the correct answer rate of the healthy elderly subjects was 66.7%, the correct answer rate of the healthy middle-aged subjects was 71.4%, and the correct answer rate of the healthy young subjects was 90.0%.

For the same Alzheimer-type dementia patients using the heart rate data acquired at the date and time more than 4 months before, the correct answer rate of judgement based on the predicted amplitude ratio of the sine wave and the cosine wave of the circadian rhythm and the non-circadian rhythm according to the first embodiment was 57.9%. The reason for this is thought to be that the coefficients of the estimated heart rate cancel each other out as described above because of light sleep due to the summer season. Therefore, as a result of evaluation by the method according to the present embodiment, the correct answer rate for Alzheimer-type dementia patients became 82.4%, meaning that the correct answer rate was improved.

As described above, in the present embodiment, it is judged whether the subject has Alzheimer-type dementia according to the instability ratio of circadian rhythm waves estimated from heart rate data.

Thus, according to the present embodiment, it is possible to accurately judge whether the subject is an Alzheimer-type dementia patient. In the present embodiment, the evaluation results shows that the Alzheimer-type dementia judgement rate is high both in the month when the progress of Alzheimer-type dementia in its patients is suspected to be significant and several months before that month.

Here, in the above example, the example in which heart rate data is employed as biological information has been described, but the biological information can be, for example, pulse rate data. Here, in the embodiment, "information about a heart rate" corresponds to "heart rate data" or "pulse rate data." In the above example, the example in which the instability of circadian rhythm waves is calculated by the ratio of the coefficient has been described, but the present invention is not limited thereto. The judgement unit 15A may distinguish between Alzheimer-type dementia patients and healthy subjects using the instability of circadian rhythm waves based on the difference between the sum of absolute values of the coefficients and the sum of the coefficients.

<Third embodiment>

In the second embodiment, the example in which the instability of circadian rhythm waves is calculated by the coefficient of the estimated heart rate has been described. The cosine wave is sensitive to the wave instability, and healthy subjects may be erroneously judged. Therefore, in the present embodiment, an example in which the instability is judged using the sine wave component of the estimated heart rate will be described.

(Overview of method according to present embodiment)

The estimated heart rate $f_{CR}(t)$ has a large amount of variation in the coefficient of the cosine wave because the heart rate gradually decreases as the sleep becomes deeper after the data start time. However, when the amount of variation in the sine wave increases toward the latter half of the data, there is a trend to update the coefficients of the cosine wave up to that point in a suppressing or canceling manner. At this time, it is evaluated as unstable even if a circadian variation is observed in the heart rate.

In such a case, there is a high possibility of healthy subjects being judged normally according to the amplitude of the circadian rhythm itself rather than the instability of the circadian rhythm. That is, it is better to switch the method of the second embodiment to the method of the first embodiment.

Therefore, in the present embodiment, when the sine wave is evaluated as stable and the cosine wave is evaluated as unstable according to judgement based on the method of the second embodiment, it is switched to judgement based on the method of the first embodiment.

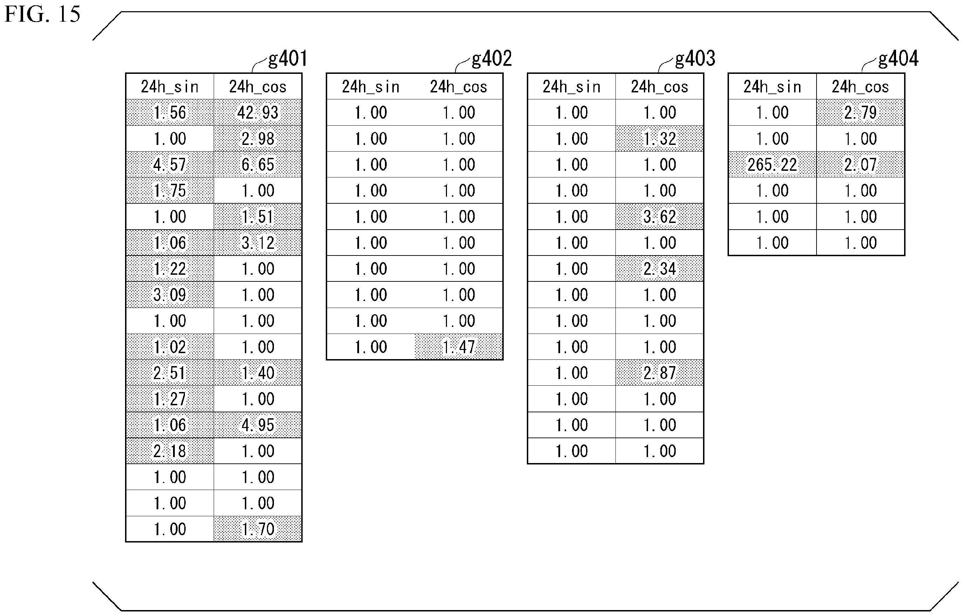

FIG. 15 is the diagram showing the examples of the instability ratio of the sine wave and the cosine wave of the Alzheimer-type dementia patients, the healthy young subjects, the healthy middle-aged subjects, and the healthy elderly subjects. Table g401 shows the instability ratio of the sine wave and the cosine wave of the Alzheimer-type dementia patients. Table g402 shows the instability ratio of the sine wave and the cosine wave of the healthy young subjects. Table g403 shows the instability ratio of the sine wave and the cosine wave of the healthy middle-aged subjects. Table g404 shows the instability ratio of the sine wave and the cosine wave of the healthy elderly subjects. In Tables g401 to g404, the left column indicates the instability ratio of the sine wave while the right column indicates the instability ratio of the cosine wave. In Tables g401 to g404, the hatched columns indicate that the wave instability ratio is larger than 1 (i.e., the wave is unstable).

As shown in Tables g401 to g404, generally, the wave instability ratio of the Alzheimer-type dementia patients is larger than the wave instability ratio of the healthy subjects. Here, as shown in Tables g401 to g404, the instability ratio of the sine wave of the Alzheimer-type dementia patients is larger than the wave instability ratio of the healthy subjects.

Therefore, in the present embodiment, it is judged whether the subject is an Alzheimer-type dementia patient according to the instability ratio of the sine wave in the estimated heart rate.

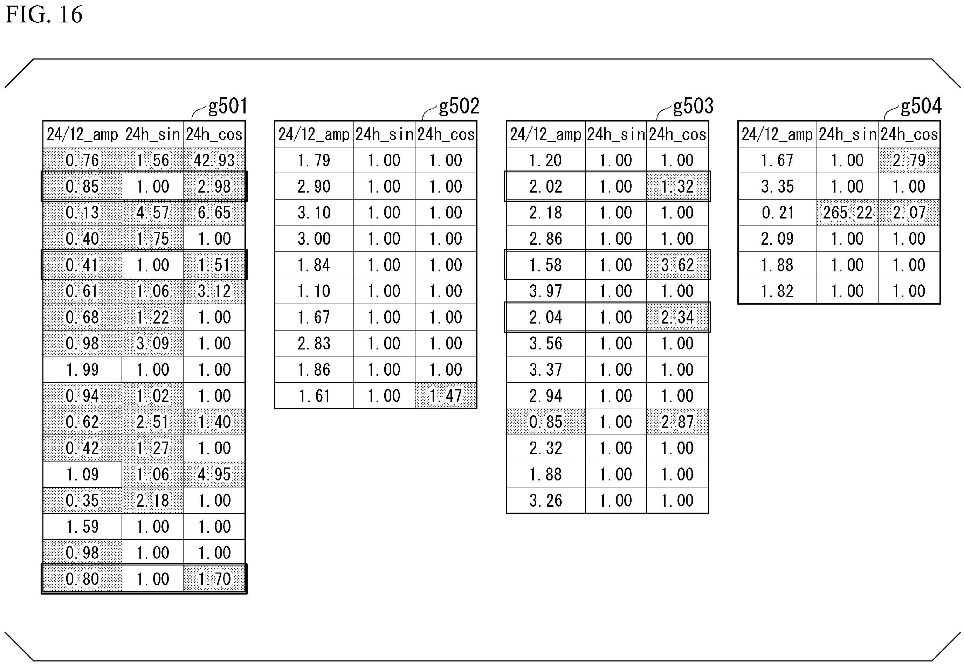

FIG. 16 is the diagram for illustrating the method according to the present embodiment. Table g501 shows the predicted amplitude ratio of the circadian rhythm and the non-circadian rhythm, and the instability ratio of the sine wave and the cosine wave of the Alzheimer-type dementia patients. Table g502 shows the predicted amplitude ratio of the circadian rhythm and the non-circadian rhythm, and the instability ratio of the sine wave and the cosine wave of the healthy young subjects. Table g503 shows the predicted amplitude ratio of the circadian rhythm and the non-circadian rhythm, and the instability ratio of the sine wave and the cosine wave of the healthy middle-aged subjects. Table g504 shows the predicted amplitude ratio of the circadian rhythm and the non-circadian rhythm and the instability ratio of the sine wave and the cosine wave of the healthy elderly subjects. In Tables g501 to g504, the left column indicates the predicted amplitude ratio of the circadian rhythm and the non-circadian rhythm, the middle column indicates the instability ratio of the sine wave, and the right column indicates the instability ratio of the cosine wave. In Tables g501 to g504, the hatched columns in the predicted amplitude ratio of the circadian rhythm and the non-circadian rhythm indicate that the ratio is smaller than 1 (i.e., the wave is unstable), and the hatched columns in the instability ratio of the sine wave and the cosine wave indicate that the ratio is larger than 1 (i.e., the wave is unstable). Tables g501 to g504 show that, particularly, the instability ratio of the sine wave of the Alzheimer-type dementia patients is larger than the instability ratio of the sine wave of the healthy subjects.

Here, in the present embodiment, the judgement unit 15C judges that the subject is an Alzheimer-type dementia patient when the instability ratio of the coefficient of the sine wave is larger than 1, and judges that the subject is healthy when the instability ratio of the coefficient of the sine wave is 1. In the present embodiment, when the instability ratio of the coefficient of the sine wave is 1 and the instability ratio of the coefficient of the cosine wave is larger than 1, the method of the first embodiment is employed for judgement.

[Configuration of dementia judgement device]

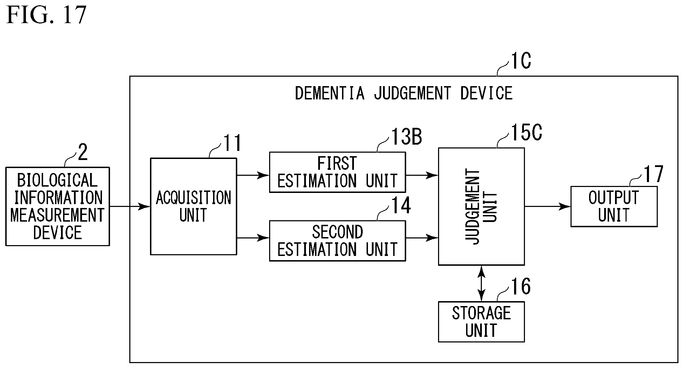

FIG. 17 is the diagram showing the configuration example of a dementia judgement device according to the present embodiment. As shown in FIG. 17, the dementia judgement device 1C includes the acquisition unit 11, the first estimation unit 13B, the second estimation unit 14, the judgement unit 15C, the storage unit 16, and the output unit 17.

During measurement, the biological information measurement device 2 is connected to the dementia judgement device 1C.

The judgement unit 15C judges that the subject is an Alzheimer-type dementia patient when the instability ratio of the coefficient of the sine wave is larger than 1, and judges that the subject is healthy when the instability ratio of the coefficient of the sine wave is 1. When the instability ratio of the coefficient of the sine wave is 1 and the instability ratio of the coefficient of the cosine wave is 1, the judgement unit 15C performs judgement through a comparison the ratio of the amplitude of the circadian rhythm and the non-circadian rhythm with the threshold value.

Next, the processing procedure example of the dementia judgement device will be described.

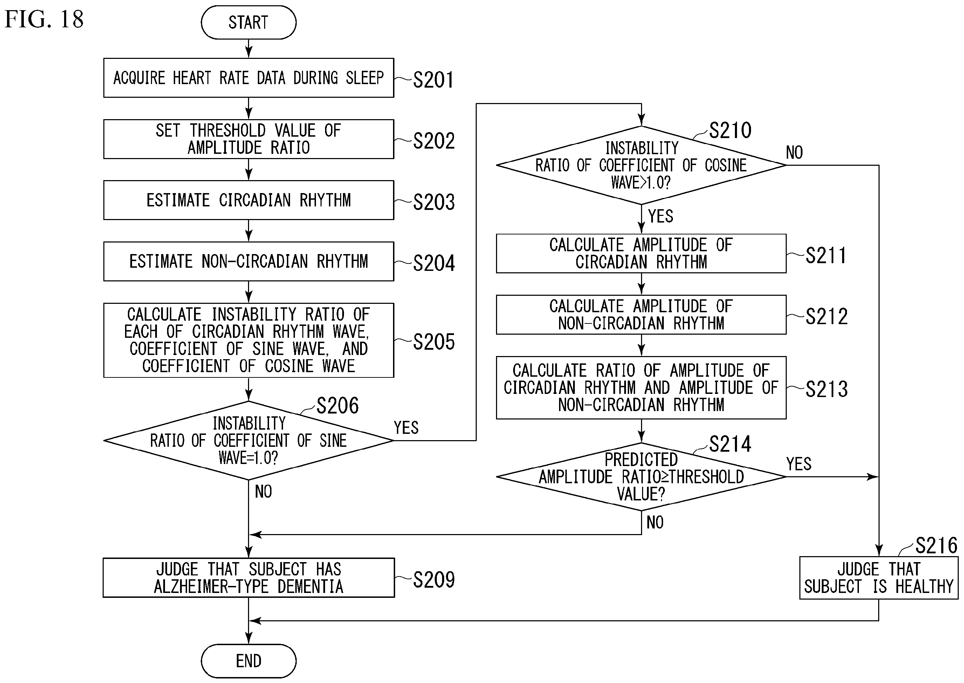

FIG. 18 is the flowchart of processing procedures of the dementia judgement device according to the present embodiment.

(Step S201) The acquisition unit 11 acquires heart rate data during sleep.

(Step S202) The judgement unit 15C sets a threshold value $r_{amp}$ of the amplitude ratio.

(Step S203) The first estimation unit 13B estimates a circadian rhythm from the heart rate data.

(Step S204) The second estimation unit 14 estimates a non-circadian rhythm from the heart rate data.

(Step S205) The judgement unit 15C calculates an instability ratio R of circadian rhythm waves. The judgement unit 15C acquires a coefficient of the sine wave and a coefficient of the cosine wave of circadian rhythm waves. That is, the judgement unit 15C calculates the ratio of the sum of absolute values of the coefficients of the sine wave component of the estimated circadian rhythm of the heart rate and the sum of the coefficients of the sine wave component of the estimated circadian rhythm of the heart rate as the instability ratio $R_{sin}$ of the sine wave. In the same way, the judgement unit 15C calculates the ratio of the sum of absolute values of the coefficients of the cosine wave component of the estimated circadian rhythm of the heart rate and the sum of the coefficients of the cosine component of the estimated circadian rhythm of the heart rate as the instability ratio $R_{cos}$ of the sine wave.

(Step S206) The judgement unit 15C judges whether the instability ratio $R_{sin}$ of the coefficient of the sine wave is 1.0. When the determination unit 15C determines that the instability ratio $R_{sin}$ of the coefficient of the sine wave is 1.0 (YES in Step S206), the process proceeds to Step S210. When the determination unit 15C determines that the

instability ratio $R_{sin}$ of the coefficient of the sine wave is not 1.0 (NO in Step S206), the process proceeds to Step S209.

(Step S210) The judgement unit 15C judges whether the instability ratio $R_{cos}$ of the coefficient of the cosine wave is larger than 1.0. When the determination unit 15C determines that the instability ratio $R_{cos}$ of the coefficient of the cosine wave is larger than 1.0 (YES in Step S210), the process proceeds to Step S211. When the determination unit 15C determines that the instability ratio $R_{cos}$ of the coefficient of the cosine wave is 1.0 or less (NO in Step S216), the process proceeds to Step S209.

(Step S211) The judgement unit 15C calculates a signal amplitude of the estimated circadian rhythm.

(Step S212) The judgement unit 15C calculates a signal amplitude of the estimated non-circadian rhythm.

(Step S213) The judgement unit 15C calculates a predicted amplitude ratio, which is a ratio of the signal amplitude of the circadian rhythm and the non-circadian rhythm.

(Step S214) The judgement unit 15C judges whether the predicted amplitude ratio is equal to or larger than the threshold value $r_{amp}$ (for example, the threshold value $r_{amp}$ is 1.0). When the determination unit 15C determines that the predicted amplitude ratio is equal to or larger than the threshold value $r_{amp}$ (YES in Step S214), the process proceeds to Step S216. When the determination unit 15C determines that the predicted amplitude ratio is less than the threshold value $r_{amp}$ (NO in Step S214), the process proceeds to Step S209.

(Step S209) The judgement unit 15C judges that the subject is an Alzheimer-type dementia patient when the instability ratio $R_{sin}$ of the coefficient of the sine wave is not 1.0 or the predicted amplitude ratio is less than the threshold value $r_{amp}$. After the process, the judgement unit 15C terminates the process.

(Step S216) The judgement unit 15C judges that the subject is healthy when the ratio is equal to or larger than the threshold value. After the process, the judgement unit 15C terminates the process.

Here, in the above process, the judgement unit 15C may calculate an instability ratio $R_{sin}$ of the coefficient of the sine wave in the circadian rhythm to judge whether the subject is an Alzheimer-type dementia based on this value. In the case, in the process, the acquisition unit acquires heart rate data, and the judgement unit 15C calculates the instability ratio $R_{sin}$ of the sine wave of circadian rhythm waves. Then, the judgement unit 15C may judge whether the instability ratio of the coefficient of the sine wave is 1.0. When the instability ratio of the coefficient of the sine wave is 1.0, the judgement unit 15C may judge that the subject is healthy. When the instability ratio of the coefficient of the sine wave is not 1.0, the judgement unit 15C may judge that the subject is an Alzheimer-type dementia patient.

As described above, in the present embodiment, the acquisition unit 11 acquires information about a heart rate, the first estimation unit 13B estimates a circadian rhythm of the heart rate from the information about the heart rate, and the judgement unit 15C judges whether the subject has Alzheimer-type dementia based on the sine wave component of the signal of the estimated circadian rhythm of the heart rate. , in the present embodiment, the judgement unit 15C judges whether the subject has Alzheimer-type dementia additionally using the cosine wave component of the signal of the estimated circadian rhythm of the heart rate and the predicted amplitude ratio.

[Evaluation results]

Next, the evaluation result example will be described. Here, in the evaluation, the human subject experiment was approved by the ethics committee institution to which the inventors belong, and all subjects signed the consent form.

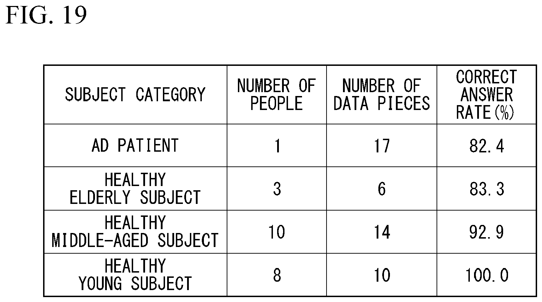

FIG. 19 is the diagram showing examples of evaluation results by the method according to the present embodiment. As shown in FIG. 17, according to the present embodiment, the correct answer rate for the Alzheimer-type dementia patients was maintained at 82.4%, the correct answer rate for the healthy elderly subjects was 83.3%, the correct answer rate for the healthy middle-aged subjects was 92.9%, and the correct answer rate for the healthy young subjects could be improved to 100.0%.

As a result of evaluation, there were a total of 4 cases of data from the Alzheimer-type dementia patients with an $R_{sin}$ of 1.0 and an $R_{cos}$ of larger than 1.0, and a total of 5 cases of data from the healthy subjects. Among these, the results of comparison with the threshold value $r_{amp}$ of the amplitude ratio were a total of 0 cases of data from the Alzheimer-type dementia patients and a total of 4 cases of data from the healthy subjects. That is, according to the present embodiment, it is possible to improve the healthy subject judgement accuracy without affecting Alzheimer-type dementia judgement accuracy.

As described above, in the present embodiment, it is judged whether the subject is an Alzheimer-type dementia patient by the instability ratio of the coefficient of the cosine wave and the ratio of the signal amplitude of the circadian rhythm and the non-circadian rhythm, in addition to the instability ratio of the coefficient of the sine wave.

Thus, according to the present embodiment, it is possible to reduce erroneous judgement (false positive) of healthy subjects.

Here, in the above example, the example in which heart rate data is employed as biological information has been described, but the biological information can be, for example, pulse rate data. Here, in the embodiment, "information about a heart rate" corresponds to "heart rate data" or "pulse rate data." In the above example, the example in which the instability of circadian rhythm waves is calculated by the ratio of the coefficients of the sine wave has been described, but the present invention is not limited thereto. The judgement unit 15C may distinguish between Alzheimer-type dementia patients and healthy subjects according to the instability of circadian rhythm waves based on the difference between the sum of absolute values of the coefficients of the sine wave and the sum of the coefficients of the sine wave.

Here, the program for implementing all or some functions of the dementia judgement device 1 (or 1A, 1B, 1C) in the present invention is recorded in a computer readable recording medium, and a computer system reads and executes the program recorded in the recording medium, and thus all or some processes performed by the dementia judgement device 1 (or 1A, 1B, 1C) may be performed. The term "computer system" used herein includes an OS or hardware such as peripheral devices. The "computer system" also includes a WWW system having a homepage providing environment (or a display environment). Moreover, the "computer readable recording media" include portable media such as a flexible disk, a magneto-optical disc, a ROM, and a CD-ROM, and a storage device built in the computer system such as a hard disk. Further, the "computer readable recording media" include media that maintain a program for a prejudged time like a volatile memory (RAM) in the computer system serving as a server or a client when the program is transmitted through a network such as the Internet or a communication line such as a telephone line.

The above program may be transmitted to another computer system from a computer system in which the program is stored in a storage device through a transmission medium or carrier waves in the transmission medium. Here, the "transmission medium" configured to transmit the program refers to a medium having a function of transmitting information such as a network (a communication network) such as the Internet or a communication line (a communication wire) such as a telephone line. The program may implement some of the above functions. Further, the above-described functions may also be implemented in combination with a program already stored in the computer system, which is a so-called differential file (differential program).

While forms for implementing the present invention have been described above with reference to embodiments, the present invention is not limited to the embodiments at all, and various modifications and substitutions can be made without departing from the spirit and scope of the present invention.

[Reference Signs List]

1, 1A, 1B, 1C Dementia judgement device
2 Biological information measurement device
11 Acquisition unit
13, 13B First estimation unit
14 Second estimation unit
15 Judgement unit
16 Storage unit
17 Output unit

**Claims**

1. An Alzheimer-type dementia judgement device, comprising:

   an acquisition unit configured to acquire information about a heart rate;
   a first estimation unit configured to estimate a circadian rhythm of the heart rate from the information about the heart rate;
   a second estimation unit configured to estimate a non-circadian rhythm of the heart rate, which is a rhythm different from the circadian rhythm of the heart rate; and
   a judgement unit configured to judge whether a subject has Alzheimer-type dementia according to a signal amplitude of the estimated circadian rhythm of the heart rate and a signal amplitude of the estimated non-circadian rhythm of the heart rate.

2. An Alzheimer-type dementia judgement device, comprising:

   an acquisition unit configured to acquire information about a heart rate;
   an estimation unit configured to estimate a circadian rhythm of the heart rate from the information about the heart rate; and
   a judgement unit configured to judge whether a subject has Alzheimer-type dementia according to a sine wave component and a cosine wave component of a signal of the estimated circadian rhythm of the heart rate.

3. An Alzheimer-type dementia judgement device, comprising:

an acquisition unit configured to acquire information about a heart rate;
a first estimation unit configured to estimate a circadian rhythm of the heart rate from the information about the heart rate; and
a judgement unit configured to judge whether a subject has Alzheimer-type dementia according to a sine wave component of a signal of the estimated circadian rhythm of the heart rate.

4. The Alzheimer-type dementia judgement device according to claim 3,
wherein the judgement unit judges whether a subject has Alzheimer-type dementia additionally using a cosine wave component of a signal of the estimated circadian rhythm of the heart rate and a predicted amplitude ratio.

5. The Alzheimer-type dementia judgement device according to claim 1,
wherein the judgement unit judges whether a subject has Alzheimer-type dementia comparing of a ratio or difference between a signal amplitude of the estimated circadian rhythm of the heart rate and a signal amplitude of the estimated non-circadian rhythm of the heart rate with a threshold value.

6. The Alzheimer-type dementia judgement device according to claim 2,
wherein the judgement unit judges whether a subject has Alzheimer-type dementia by comparing a ratio or difference between a sum of absolute values of coefficients of the estimated circadian rhythm of the heart rate and a sum of coefficients of the estimated circadian rhythm of the heart rate with a threshold value.

7. The Alzheimer-type dementia judgement device according to claim 4, further comprising

a second estimation unit configured to estimate a non-circadian rhythm of the heart rate, which is a rhythm different from the circadian rhythm of the heart rate,
wherein the judgement unit calculates an instability ratio of the sine wave as a ratio of a sum of absolute values of coefficients of a sine wave component of the estimated circadian rhythm of the heart rate and a sum of coefficients of a sine wave component of the estimated circadian rhythm of the heart rate,
calculates an instability ratio of a cosine wave as a ratio of a sum of absolute values of coefficients of a cosine wave component of the estimated circadian rhythm of the heart rate and a sum of coefficients of a cosine component of the estimated circadian rhythm of the heart rate,
judges that a subject has Alzheimer-type dementia when an instability ratio of the sine wave of the circadian rhythm of the heart rate is larger than 1,
judges that a subject is healthy when the instability ratio of the sine wave of the circadian rhythm of the heart rate is 1 and the instability ratio of the sine wave of the circadian rhythm of the heart rate is 1 or less,
judges that a subject has Alzheimer-type dementia when the instability ratio of the sine wave of the circadian rhythm of the heart rate is 1 and the instability ratio of the cosine wave of the circadian rhythm of the heart rate is larger than 1 and when a ratio of a signal amplitude of the estimated circadian rhythm of the heart rate and a signal amplitude of the estimated non-circadian rhythm of the heart rate is smaller than a threshold value, and
judges that a subject is healthy when the instability ratio of the sine wave of the circadian rhythm of the heart rate is 1 or less or the instability ratio of the cosine wave of the circadian rhythm of the heart rate is larger than 1 and when a ratio of a signal amplitude of the estimated circadian rhythm of the heart rate and a signal amplitude of the estimated non-circadian rhythm of the heart rate is equal to or larger than a threshold value.

8. An Alzheimer-type dementia judgement method, comprising:

acquiring, by an acquisition unit, information about a heart rate;
estimating, by a first estimation unit, a circadian rhythm of the heart rate from the information about the heart rate;
estimating, by a second estimation unit, a non-circadian rhythm of the heart rate, which is a rhythm different from the circadian rhythm of the heart rate; and
determining, by a judgement unit, whether a subject has Alzheimer-type dementia according to a signal amplitude of the estimated circadian rhythm of the heart rate and a signal amplitude of the estimated non-circadian rhythm of the heart rate.

9. An Alzheimer-type dementia judgement method, comprising:

acquiring, by an acquisition unit, information about a heart rate;

estimating, by an estimation unit, a circadian rhythm of the heart rate from the information about the heart rate; and

determining, by a judgement unit, whether a subject has Alzheimer-type dementia according to a sine wave component and a cosine wave component of a signal of the estimated circadian rhythm of the heart rate.

10. An Alzheimer-type dementia judgement method, comprising:

acquiring, by an acquisition unit, information about a heart rate;

estimating, by a first estimation unit, a circadian rhythm of the heart rate from the information about the heart rate; and

determining, by a judgement unit, whether a subject has Alzheimer-type dementia according to a sine wave component of a signal of the estimated circadian rhythm of the heart rate.

11. A program causing a computer to:

acquire information about a heart rate;

estimate a circadian rhythm of the heart rate from the information about the heart rate;

estimate a non-circadian rhythm of the heart rate, which is a rhythm different from the circadian rhythm; and

judge whether a subject has Alzheimer-type dementia according to a signal amplitude of the estimated circadian rhythm of the heart rate and a signal amplitude of the estimated non-circadian rhythm of the heart rate.

12. A program causing a computer to:

acquire information about a heart rate;

estimate a circadian rhythm of the heart rate from the information about the heart rate; and

judge whether a subject has Alzheimer-type dementia according to a sine wave component and a cosine wave component of a signal of the estimated circadian rhythm of the heart rate.

13. A program causing a computer to:

acquire information about a heart rate;

estimate a circadian rhythm of the heart rate from the information about the heart rate; and

judge whether a subject has Alzheimer-type dementia according to a sine wave component of a signal of the estimated circadian rhythm of the heart rate.

FIG. 1

FIG. 2

FIG. 3

EP 4 302 704 A1

FIG. 4

FIG. 5

EP 4 302 704 A1

FIG. 6

```
                    ┌─────────────┐
                    │    START    │
                    └──────┬──────┘
                           ▼
          ┌─────────────────────────────┐
          │   ACQUIRE HEART RATE DATA    │  ～S1
          │        DURING SLEEP          │
          └──────────────┬──────────────┘
                         ▼
          ┌─────────────────────────────┐
          │     SET THRESHOLD VALUE OF   │  ～S2
          │        AMPLITUDE RATIO       │
          └──────────────┬──────────────┘
                         ▼
          ┌─────────────────────────────┐
          │   ESTIMATE CIRCADIAN RHYTHM  │  ～S3
          └──────────────┬──────────────┘
                         ▼
          ┌─────────────────────────────┐
          │ ESTIMATE NON-CIRCADIAN RHYTHM│  ～S4
          └──────────────┬──────────────┘
                         ▼
          ┌─────────────────────────────┐
          │      CALCULATE AMPLITUDE OF  │  ～S5
          │        CIRCADIAN RHYTHM      │
          └──────────────┬──────────────┘
                         ▼
          ┌─────────────────────────────┐
          │      CALCULATE AMPLITUDE OF  │  ～S6
          │      NON-CIRCADIAN RHYTHM    │
          └──────────────┬──────────────┘
                         ▼
          ┌─────────────────────────────┐
          │ CALCULATE RATIO OF AMPLITUDE OF│ ～S7
          │ CIRCADIAN RHYTHM AND AMPLITUDE │
          │  OF NON-CIRCADIAN RHYTHM.     │
          └──────────────┬──────────────┘
```

～S8

PREDICTED
AMPLITUDE RATIO≥THRESHOLD
VALUE?

NO

YES

～S9
JUDGE THAT
SUBJECT IS HEALTHY

～S10
JUDGE THAT SUBJECT IS
ALZHEIMER-TYPE DEMENTIA PATIENT

END

FIG. 7

g210

g200

FIG. 8

FIG. 9

| SUBJECT CATEGORY | NUMBER OF PEOPLE | NUMBER OF DATA PIECES | CORRECT ANSWER RATE (%) |
|---|---|---|---|
| AD PATIENT | 1 | 17 | 82.4 |
| HEALTHY ELDERLY SUBJECT | 3 | 6 | 83.3 |
| HEALTHY MIDDLE-AGED SUBJECT | 10 | 14 | 92.9 |
| HEALTHY YOUNG SUBJECT | 8 | 10 | 100.0 |

FIG. 10

EP 4 302 704 A1

FIG. 11

FIG. 12

## FIG. 13

START

ACQUIRE HEART RATE
DATA DURING SLEEP — S1

ESTIMATE CIRCADIAN RHYTHM — S2

CALCULATE INSTABILITY RATIO OF
CIRCADIAN RHYTHM WAVE — S101

S102

INSTABILITY RATIO=1.0?  NO

YES

JUDGE THAT SUBJECT IS
HEALTHY — S103

JUDGE THAT SUBJECT IS
ALZHEIMER-TYPE DEMENTIA PATIENT — S104

END

## FIG. 14

| SUBJECT CATEGORY | NUMBER OF PEOPLE | NUMBER OF DATA PIECES | CORRECT ANSWER RATE (%) |
|---|---|---|---|
| AD PATIENT | 1 | 17 | 82.4 |
| HEALTHY ELDERLY SUBJECT | 3 | 6 | 66.7 |
| HEALTHY MIDDLE-AGED SUBJECT | 10 | 14 | 71.4 |
| HEALTHY YOUNG SUBJECT | 8 | 10 | 90.0 |

**FIG. 15**

g401

| 24h_sin | 24h_cos |
|---|---|
| 1.56 | 42.93 |
| 1.00 | 2.98 |
| 4.57 | 6.65 |
| 1.75 | 1.00 |
| 1.00 | 1.51 |
| 1.06 | 3.12 |
| 1.22 | 1.00 |
| 3.09 | 1.00 |
| 1.00 | 1.00 |
| 1.02 | 1.00 |
| 2.51 | 1.40 |
| 1.27 | 1.00 |
| 1.06 | 4.95 |
| 2.18 | 1.00 |
| 1.00 | 1.00 |
| 1.00 | 1.00 |
| 1.00 | 1.70 |

g402

| 24h_sin | 24h_cos |
|---|---|
| 1.00 | 1.00 |
| 1.00 | 1.00 |
| 1.00 | 1.00 |
| 1.00 | 1.00 |
| 1.00 | 1.00 |
| 1.00 | 1.00 |
| 1.00 | 1.00 |
| 1.00 | 1.00 |
| 1.00 | 1.00 |
| 1.00 | 1.47 |

g403

| 24h_sin | 24h_cos |
|---|---|
| 1.00 | 1.00 |
| 1.00 | 1.32 |
| 1.00 | 1.00 |
| 1.00 | 1.00 |
| 1.00 | 3.62 |
| 1.00 | 1.00 |
| 1.00 | 2.34 |
| 1.00 | 1.00 |
| 1.00 | 1.00 |
| 1.00 | 1.00 |
| 1.00 | 2.87 |
| 1.00 | 1.00 |
| 1.00 | 1.00 |
| 1.00 | 1.00 |

g404

| 24h_sin | 24h_cos |
|---|---|
| 1.00 | 2.79 |
| 1.00 | 1.00 |
| 265.22 | 2.07 |
| 1.00 | 1.00 |
| 1.00 | 1.00 |
| 1.00 | 1.00 |

EP 4 302 704 A1

## FIG. 16

g501

| 24/12_amp | 24h_sin | 24h_cos |
|---|---|---|
| 0.76 | 1.56 | 42.93 |
| 0.85 | 1.00 | 2.98 |
| 0.13 | 4.57 | 6.65 |
| 0.40 | 1.75 | 1.00 |
| 0.41 | 1.00 | 1.51 |
| 0.61 | 1.06 | 3.12 |
| 0.68 | 1.22 | 1.00 |
| 0.98 | 3.09 | 1.00 |
| 1.99 | 1.00 | 1.00 |
| 0.94 | 1.02 | 1.00 |
| 0.62 | 2.51 | 1.40 |
| 0.42 | 1.27 | 1.00 |
| 1.09 | 1.06 | 4.95 |
| 0.35 | 2.18 | 1.00 |
| 1.59 | 1.00 | 1.00 |
| 0.98 | 1.00 | 1.00 |
| 0.80 | 1.00 | 1.70 |

g502

| 24/12_amp | 24h_sin | 24h_cos |
|---|---|---|
| 1.79 | 1.00 | 1.00 |
| 2.90 | 1.00 | 1.00 |
| 3.10 | 1.00 | 1.00 |
| 3.00 | 1.00 | 1.00 |
| 1.84 | 1.00 | 1.00 |
| 1.10 | 1.00 | 1.00 |
| 1.67 | 1.00 | 1.00 |
| 2.83 | 1.00 | 1.00 |
| 1.86 | 1.00 | 1.00 |
| 1.61 | 1.00 | 1.47 |

g503

| 24/12_amp | 24h_sin | 24h_cos |
|---|---|---|
| 1.20 | 1.00 | 1.00 |
| 2.02 | 1.00 | 1.32 |
| 2.18 | 1.00 | 1.00 |
| 2.86 | 1.00 | 1.00 |
| 1.58 | 1.00 | 3.62 |
| 3.97 | 1.00 | 1.00 |
| 2.04 | 1.00 | 2.34 |
| 3.56 | 1.00 | 1.00 |
| 3.37 | 1.00 | 1.00 |
| 2.94 | 1.00 | 1.00 |
| 0.85 | 1.00 | 2.87 |
| 2.32 | 1.00 | 1.00 |
| 1.88 | 1.00 | 1.00 |
| 3.26 | 1.00 | 1.00 |

g504

| 24/12_amp | 24h_sin | 24h_cos |
|---|---|---|
| 1.67 | 1.00 | 2.79 |
| 3.35 | 1.00 | 1.00 |
| 0.21 | 265.22 | 2.07 |
| 2.09 | 1.00 | 1.00 |
| 1.88 | 1.00 | 1.00 |
| 1.82 | 1.00 | 1.00 |

EP 4 302 704 A1

FIG. 17

FIG. 18

START

ACQUIRE HEART RATE DATA DURING SLEEP — S201

SET THRESHOLD VALUE OF AMPLITUDE RATIO — S202

ESTIMATE CIRCADIAN RHYTHM — S203

ESTIMATE NON-CIRCADIAN RHYTHM — S204

CALCULATE INSTABILITY RATIO OF EACH OF CIRCADIAN RHYTHM WAVE, COEFFICIENT OF SINE WAVE, AND COEFFICIENT OF COSINE WAVE — S205

S206 INSTABILITY RATIO OF COEFFICIENT OF SINE WAVE=1.0? — YES / NO

S210 INSTABILITY RATIO OF COEFFICIENT OF COSINE WAVE>1.0? — NO / YES

CALCULATE AMPLITUDE OF CIRCADIAN RHYTHM — S211

CALCULATE AMPLITUDE OF NON-CIRCADIAN RHYTHM — S212

CALCULATE RATIO OF AMPLITUDE OF CIRCADIAN RHYTHM AND AMPLITUDE OF NON-CIRCADIAN RHYTHM — S213

S214 PREDICTED AMPLITUDE RATIO≥THRESHOLD VALUE? — YES / NO

JUDGE THAT SUBJECT HAS ALZHEIMER-TYPE DEMENTIA — S209

JUDGE THAT SUBJECT IS HEALTHY — S216

END

FIG. 19

| SUBJECT CATEGORY | NUMBER OF PEOPLE | NUMBER OF DATA PIECES | CORRECT ANSWER RATE (%) |
|---|---|---|---|
| AD PATIENT | 1 | 17 | 82.4 |
| HEALTHY ELDERLY SUBJECT | 3 | 6 | 83.3 |
| HEALTHY MIDDLE-AGED SUBJECT | 10 | 14 | 92.9 |
| HEALTHY YOUNG SUBJECT | 8 | 10 | 100.0 |

## INTERNATIONAL SEARCH REPORT

| International application No. |
|---|
| **PCT/JP2022/008871** |

**A. CLASSIFICATION OF SUBJECT MATTER**

*A61B 10/00*(2006.01)i; *A61B 5/0245*(2006.01)i; *A61B 5/11*(2006.01)i
FI: A61B10/00 H; A61B5/11 100; A61B5/0245 A

According to International Patent Classification (IPC) or to both national classification and IPC

**B. FIELDS SEARCHED**

Minimum documentation searched (classification system followed by classification symbols)

A61B5/00-5/398; A61B10/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); Scopus

**C. DOCUMENTS CONSIDERED TO BE RELEVANT**

| Category* | Citation of document, with indication, where appropriate, of the relevant passages | Relevant to claim No. |
|---|---|---|
| A | US 2002/156392 A1 (MITSUBISHI CHEMICAL CORP.) 24 October 2002 (2002-10-24) entire text, all drawings | 1-13 |
| A | 納戸美佐子ほか, 認知症高齢者の睡眠時・覚醒時における心拍変動に関する継時的変化, 久留米大学文学部紀要 社会福祉学科編, 2007, vol. 7, pp. 127-134 (NOTO, Misako. Long-term change of heart rate variability when sleep-wake in elderly persons with dementia. Bulletin of Faculty of Literature, Kurume University, Social Welfare.) in particular, pp. 131, 132 | 1-13 |
| A | 鈴木圭輔ほか, 不眠症と生体リズム 神経変性疾患と生体リズム, ねむりと医療, 2013, vol. 6, no. 1, pp. 12-17, ISSN: 1883-0552 (SUZUKI, Keisuke et al. Insomnia and Biological Rhythm: Neurodegenerative Diseases and Biological Rhythm. Sleep and Clinical Practice.) In particular, "Alzeheimer's Disease" | 1-13 |
| A | 島本順子ほか, 老年者における痴呆及び寝たきり状態が心拍数概日リズムに及ぼす影響, 日本老年医学会雑誌, 1988, vol. 25, no. 4, pp. 408-412, ISSN: 0300-9173 (SHIMAMOTO, Yoriko et al. The Influence of Dementia and Physical Disability on the Circadian Rhythm of Heart Rate in the Elderly. Japanese Journal of Geriatrics.) entire text | 1-13 |

☑ Further documents are listed in the continuation of Box C.   ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **15 April 2022** | **10 May 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

| | International application No. |
|---|---|
| | **PCT/JP2022/008871** |

### C.    DOCUMENTS CONSIDERED TO BE RELEVANT

| Category* | Citation of document, with indication, where appropriate, of the relevant passages | Relevant to claim No. |
|---|---|---|
| T | MATSUDA, Naoya et al., Alzheimer Dementia Detection based on Circadian Rhythm Disorder of Heartrate, Proceedings of 2021 IEEE 3rd Global Conference on Life Sciences and Technologies (LifeTech 2021), 2021, pp. 360-364, DOI: 10.1109/LifeTech52111.2021.9391871<br>   entire text | 1-13 |
| P, A | JP 2021-178174 A (KYOTO PREFECTURAL PUBLIC UNIVERSITY CORP.) 18 November 2021 (2021-11-18)<br>   entire text, all drawings | 1-13 |

Form PCT/ISA/210 (second sheet) (January 2015)

**INTERNATIONAL SEARCH REPORT**
Information on patent family members

International application No.

**PCT/JP2022/008871**

| Patent document cited in search report | | Publication date (day/month/year) | Patent family member(s) | Publication date (day/month/year) |
|---|---|---|---|---|
| US 2002/0156392 A1 | | 24 October 2002 | (Family: none) | |
| JP 2021-178174 A | | 18 November 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63156936 B

- JP 2021178089 A

**Non-patent literature cited in the description**

- **H. NIKAMALFARD et al.** A sleep pattern analysis and visualization system to support people with early dementia. *The fifth International Conference on Pervasive Computing Technologies for Healthcare and Workshops,* 2011, 510-513

- **M. MASSIN et al.** Circadian rhythm of heartrate and heartrate variability. *Archives of Disease in Child-hood,* 2000, vol. 83, 179-182
- **T. HARADA et al.** Real-Time Sleep Stage Estimation from Biological Data with Trigonometric Function Regression Model. *AAAI Spring Symposium,* 2016, 348-353